(19) **Europäisches Patentamt / European Patent Office / Office européen des brevets**

(11) **EP 4 591 856 A1**

(12) **EUROPEAN PATENT APPLICATION**
published in accordance with Art. 153(4) EPC

(43) Date of publication:
30.07.2025   Bulletin 2025/31

(21) Application number: 24781253.0

(22) Date of filing: 28.03.2024

(51) International Patent Classification (IPC):
$A61K\ 9/14$ (2006.01)     $A61K\ 35/12$ (2015.01)
$A61K\ 31/715$ (2006.01)     $A61K\ 47/10$ (2017.01)
$A61K\ 47/02$ (2006.01)     $A61P\ 17/02$ (2006.01)

(52) Cooperative Patent Classification (CPC):
A61K 9/14; A61K 31/715; A61K 35/12;
A61K 47/02; A61K 47/10; A61P 17/02

(86) International application number:
PCT/KR2024/003942

(87) International publication number:
WO 2024/205263 (03.10.2024 Gazette 2024/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.03.2023  KR 20230040173

(71) Applicants:
• POSTECH Research and Business Development
Foundation
Pohang-si, Gyeongsangbuk-do 37673 (KR)
• CJ CheilJedang Corporation
Seoul 04560 (KR)

(72) Inventors:
• HWANG, Dong Soo
Pohang-si Gyeongsangbuk-do 37673 (KR)

• OSMAN, Asila Ahmed Mohmmed
Pohang-si Gyeongsangbuk-do 37673 (KR)
• JANG, Soo Kyeong
Pohang-si Gyeongsangbuk-do 37673 (KR)
• LIN, Enhui
Pohang-si Gyeongsangbuk-do 37673 (KR)
• YI, Gi Ra
Pohang-si Gyeongsangbuk-do 37673 (KR)
• PARK, Seong Hun
Pohang-si Gyeongsangbuk-do 37673 (KR)
• SIM, Eun Jung
Seoul 04560 (KR)
• LEE, Eun Hye
Seoul 04560 (KR)
• YOON, Ki Chull
Seoul 04560 (KR)

(74) Representative: Cabinet Becker et Associés
25, rue Louis le Grand
75002 Paris (FR)

(54) **POLYMER NANOPARTICLES OF METABOLITES AND USE THEREOF**

(57)   The present disclosure relates to polymer nanoparticles of metabolites, a cell activity promotion method using same, and a cell activity promotion composition including same. In particular, the present disclosure relates to a method and a composition, for promoting, by the permeation of polymer nanoparticles of metabolites into cells, the activities of cells, for example, adhesion between cells or between tissues, hemostasis, wound healing promotion, hair root regeneration activity, and antibacterial activity.

[Fig. 1]

EP 4 591 856 A1

**Description**

**[Technical Field]**

The present disclosure relates to polymer nanoparticles of metabolites, a cell activity promotion method using same, and a cell activity promotion composition including same. Particularly, the present disclosure relates to a method and a composition for promoting cell activities, such as adhesion between cells or between tissues, hemostasis, wound healing promotion, hair root regeneration activity, and antibacterial activity by the permeation of polymer nanoparticles of metabolites into cells.

**[Background Art]**

When bleeding occurs due to injury in daily life and at industrial sites, a surgical operation is performed to prevent excessive bleeding at wound areas with rapid and safe emergency hemostasis. In the surgical operation, effective hemostasis and closure of the wound areas may reduce the amounts of bleeding and blood transfusion of a patient and promote the patient's recovery, so the treatment needs to be performed well.
Tissue adhesives used for such hemostasis and closure are rapidly receiving attention in the field of tissue or wound closure applications due to many advantages compared to conventional closure technologies, including ease of application, strong adhesion, and effective sealing against air.
However, commercially available tissue adhesive materials, such as fibrin and cyanoacrylate, have limitations such as high toxicity, weak tissue adhesion, poor mechanical strengths in wet environments, and the like. Therefore, the development of new biocompatible and highly adhesive materials that overcome these limitations is important.
In particular, in the case of trauma or chronic wounds, it is impossible to deliver nutrients into cells until nutrients are supplied from blood vessels, which need to be resolved.
However, there are limitations of currently developed hemostatic agents or medical adhesives that do not exhibit the adhesion required to suture wound areas or have low biocompatibility due to immune responses.
Meanwhile, inorganic nanoparticles have the ability to be absorbed into polymer gels due to a large surface area, and through this ability, a nanoparticle solution may serve as an adhesive to connect two hydrogels or tissues, known as a nanobridge effect. However, inorganic nanoparticles of less than 100 nm may cause inflammation, generate reactive oxygen species (ROS), or damage cell membranes through strong electrostatic interactions between nanoparticles and cells. On the other hand, organic nanoparticles are harmless to the human body and may act as nutrients only for mammalian cells. Accordingly, there is a need to develop novel materials of organic nanoparticles for nanoparticleization of biomaterials.
The present inventors have conceived that when polymer nanoparticles of metabolites (i.e., polymerized metabolite nanoparticles) adhered to cells with a large surface area and then permeated into the cells, the polymer nanoparticles rapidly supplied nutrients into the cells due to rapid decomposition into metabolites, thereby promoting cell activities, such as adhesion between cells or tissues, hemostasis, wound healing promotion, hair root regeneration activity, and antibacterial activity.

**[Disclosure]**

**[Technical Problem]**

An object of the present disclosure is to provide nanoparticles of metabolites capable of promoting cell activities, such as adhesion between cells or tissues, hemostasis, wound healing promotion, hair root regeneration activity, and antibacterial activity by permeating into cells and rapidly supplying nutrients.
Meanwhile, the technical objects to be achieved in the present disclosure are not limited to the aforementioned technical objects, and other technical objects, which are not mentioned above, will be apparently understood to those skilled in the art from the following description.

**[Technical Solution]**

In order to solve the above problem, according to an aspect of the present disclosure, there is provided a cell activity promotion composition including, as an active ingredient, polymer nanoparticles of metabolites containing at least one selected from the group consisting of peptide bonds, ester bonds, and glycosyl linkages.
Particularly, the present disclosure provides a hemostatic composition including, as an active ingredient, polymer nanoparticles of metabolites containing at least one selected from the group consisting of peptide bonds, ester bonds, and glycosyl linkages.

Further, the present disclosure provides a tissue adhesive composition including, as an active ingredient, polymer nanoparticles of metabolites containing at least one selected from the group consisting of peptide bonds, ester bonds, and glycosyl linkages.

Further, the present disclosure provides a pharmaceutical composition for promoting wound healing including, as an active ingredient, polymer nanoparticles of metabolites containing at least one selected from the group consisting of peptide bonds, ester bonds, and glycosyl linkages.

Further, the present disclosure provides an antibacterial composition including, as an active ingredient, polymer nanoparticles of metabolites containing at least one selected from the group consisting of peptide bonds, ester bonds, and glycosyl linkages.

Further, the present disclosure provides a composition for hair root regeneration including, as an active ingredient, polymer nanoparticles of metabolites containing at least one selected from the group consisting of peptide bonds, ester bonds, and glycosyl linkages.

In the hemostatic composition, the tissue adhesive composition, the pharmaceutical composition for promoting wound healing, the composition for hair root regeneration or the antibacterial composition, the polymer nanoparticles of the metabolites may be at least one selected from the group consisting of Pluronic F127, Tween20, Tween40, Tween80, gelatin, Poly(Hydroxyalkanoate) (PHA), Poly(Hydroxybutyrate) (PHB), polylactic acid (PLA), Poly(lactic-co-glycolic acid) (PLGA), levan, starch, amyloid, amyloid pectin, cellulose, chitin and chitosan.

In the hemostatic composition, the tissue adhesive composition, the pharmaceutical composition for promoting wound healing, the composition for hair root regeneration or the antibacterial composition, the polymer nanoparticles of the metabolites may have a diameter of 300 nm or less.

In the hemostatic composition, the tissue adhesive composition, the pharmaceutical composition for promoting wound healing, the composition for hair root regeneration or the antibacterial composition, the polymer nanoparticles of the metabolites may be hydrolyzed by enzymes or water within the cells after permeating into the cells to release the metabolites.

In the hemostatic composition, the tissue adhesive composition, the pharmaceutical composition for promoting wound healing, the composition for hair root regeneration or the antibacterial composition, the released metabolite may include (1) a metabolite having both a carboxyl group and a hydroxyl group, (2) a metabolite having both a carboxyl group and an amine group, (3) a metabolite having both an aldehyde group and a hydroxyl group, or (4) a metabolite having both a ketone group and a hydroxyl group.

In the hemostatic composition, the tissue adhesive composition, the pharmaceutical composition for promoting wound healing, the composition for hair root regeneration or the antibacterial composition, the released metabolite may be at least one selected from the group consisting of glucose, fructose, 3-hydroxybutyrate (3HB), 4-hydroxybutyrate (4HB), acetate, Botox and pyruvate.

Another aspect of the present disclosure provides a cell activity promotion method, including treating or administering the cell activity promotion composition according to the present disclosure to a subject other than a human or to a subject *in vitro*.

Particularly, the present disclosure provides a hemostatic method including treating or administering the hemostatic composition according to the present disclosure to a subject other than a human or to a subject *in vitro*.

Further, the present disclosure provides a tissue adhesion method including treating or administering the tissue adhesion composition according to the present disclosure to a subject other than a human or to a subject *in vitro*.

Further, the present disclosure provides a method for promoting wound healing including treating or administering the pharmaceutical composition for promoting wound healing according to the present disclosure to a subject other than a human or to a subject *in vitro*.

Further, the present disclosure provides an antibacterial method including treating or administering the antibacterial composition according to the present disclosure to a subject other than a human or to a subject *in vitro*.

Further, the present disclosure provides a method for promoting hair root regeneration or hair growth including treating or administering the composition for hair root regeneration according to the present disclosure to a subject other than a human or to a subject *in vitro*.

**[Advantageous Effects]**

According to the present disclosure, the metabolite nanoparticles permeate into cells and rapidly supply nutrients, thereby promoting cell activities, such as adhesion between cells or tissues, hemostasis, wound healing promotion, hair root regeneration activity, and antibacterial activity.

Meanwhile, effects to be achieved in the present disclosure are not limited to the aforementioned effects, and other not-mentioned effects will be obviously understood by those skilled in the art from the description below.

**[Description of Drawings]**

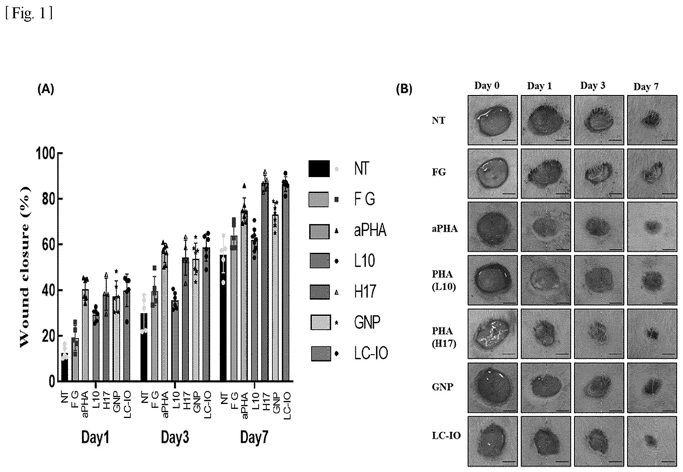

FIG. 1 shows (a) a graph showing a wound closure rate (%) and (b) an image showing the dynamics after wound healing, as a result of evaluating *in vivo* wound healing characteristics of nanoparticles synthesized from Example 1 of the present disclosure, fibrin glue (FG, positive control), and untreated wound (NT, negative control) (scale bar = 3 mm).

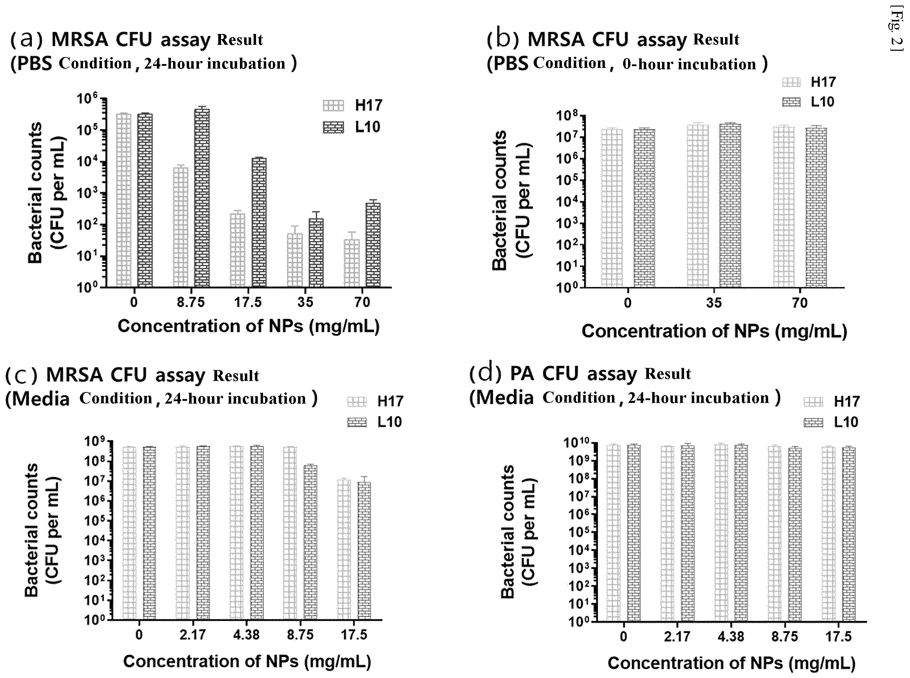

FIG. 2 shows graphs of results of *in vitro* antibacterial effect experiments, including (a) a MRSA CFU assay result (PBS conditions, 24-hour incubation), (b) a MRSA CFU assay result (PBS conditions, 0-hour incubation), (c) a MRSA CFU assay result (Media conditions, 24-hour incubation), and (c) a PA CFU assay result (Media conditions, 24-hour incubation).

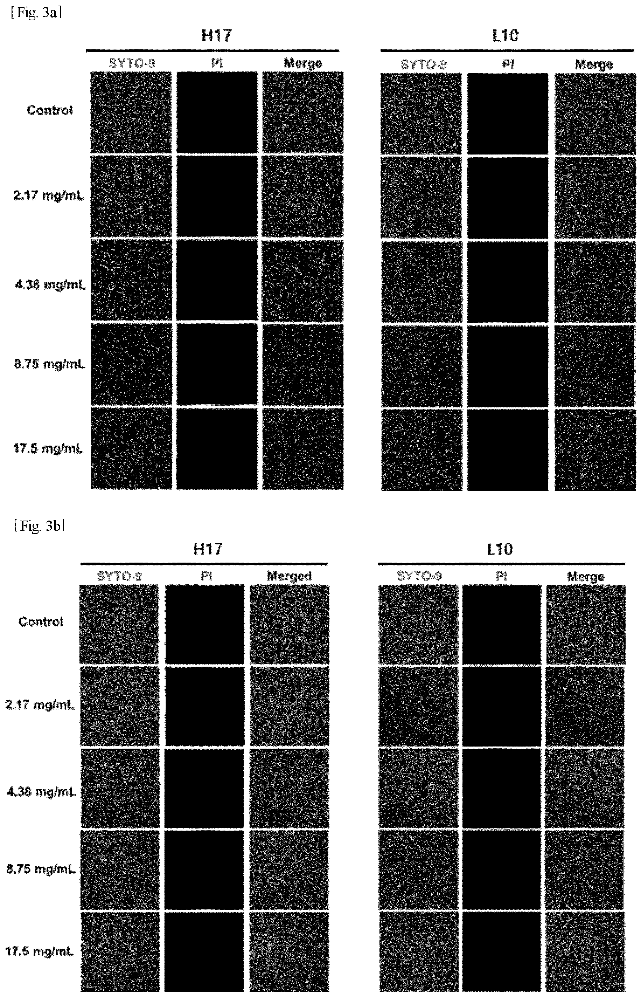

FIG. 3A is an image of an MRSA live & dead assay result, and FIG. 3B is an image of a PA live & dead assay result.

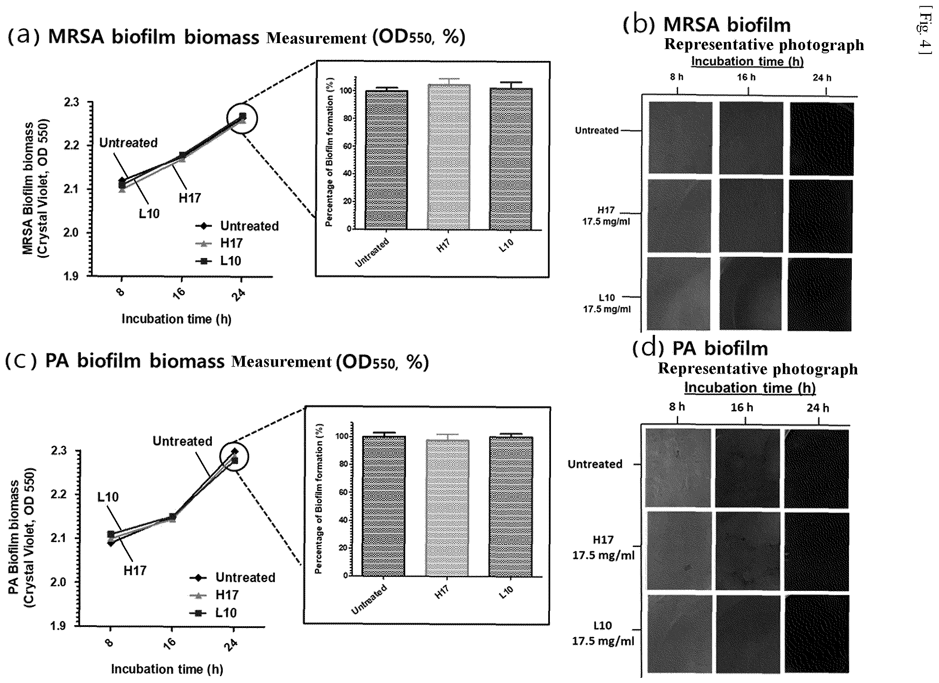

FIG. 4 shows results of biofilm eradication assay, including (a) a graph of results of measuring MRSA biofilm biomass, (b) a representative image of a MRSA biofilm, (c) a graph of results of measuring PA biofilm biomass, and (d) a representative image of a PA biofilm.

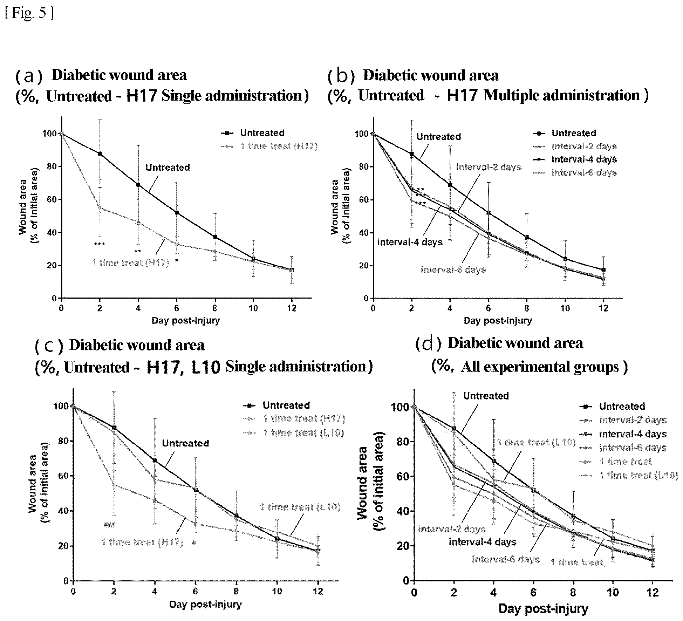

FIG. 5 shows results of an *in vivo* chronic wound healing effect experiment in a diabetes model (STZ-induced type 1 diabetes model), including (a) a graph of comparing the diabetic wound areas of an untreated group and a H17 single-administration group, (b) a graph of comparing the diabetic wound areas of an untreated group and a H17 multiple-administration group, (c) a graph of comparing the diabetic wound areas of an untreated group and a group administered with H17, L10 once, and (d) a graph of summarizing all groups in the graphs in (a) to (c) above.

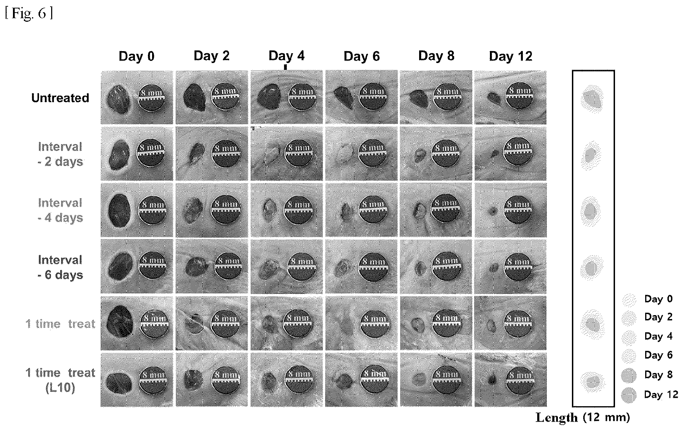

FIG. 6 is a representative image of diabetic wounds of all groups described in FIG. 5.

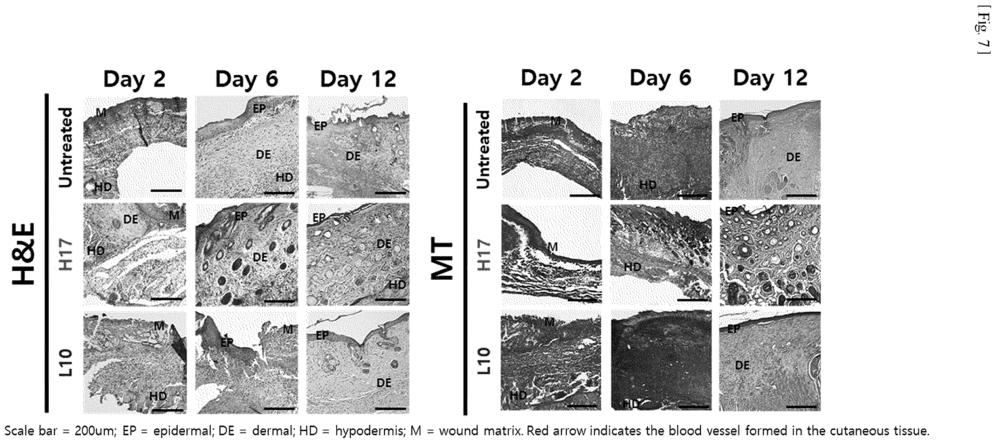

FIG. 7 shows H & E staining and MT staining images of diabetic wounds in the untreated group and the group administered with H17, L10 once described in FIG. 5, confirmed under a microscope.

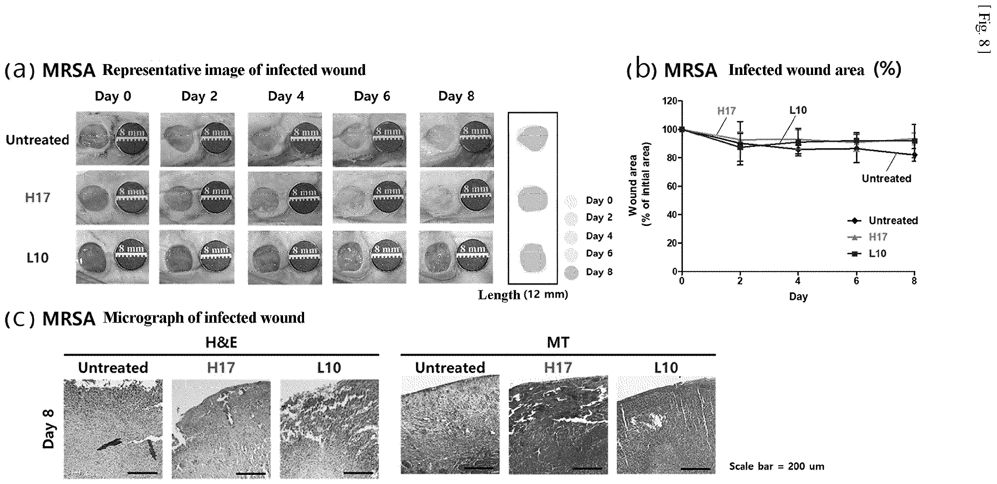

FIG. 8 shows results of an *in vivo* chronic wound healing effect experiment in a diabetes model (MRSA-infected diabetes), including (a) a representative image of a MRSA-infected wound, (b) a graph showing the area of a MRSA-infected wound, and (c) H & E staining and MT staining images of a MRSA-infected wound observed under a microscope.

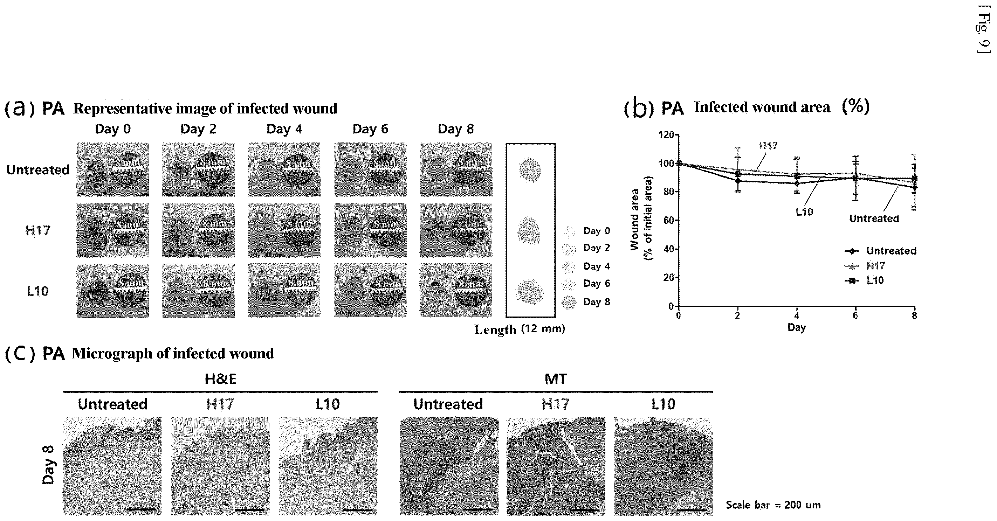

FIG. 9 shows results of an *in vivo* chronic wound healing effect experiment in a diabetes model (PA-infected diabetes model), including (a) a representative image of a PA-infected wound, (b) a graph showing the area of a PA-infected wound, and (c) H & E staining and MT staining images of a PA-infected wound observed under a microscope.

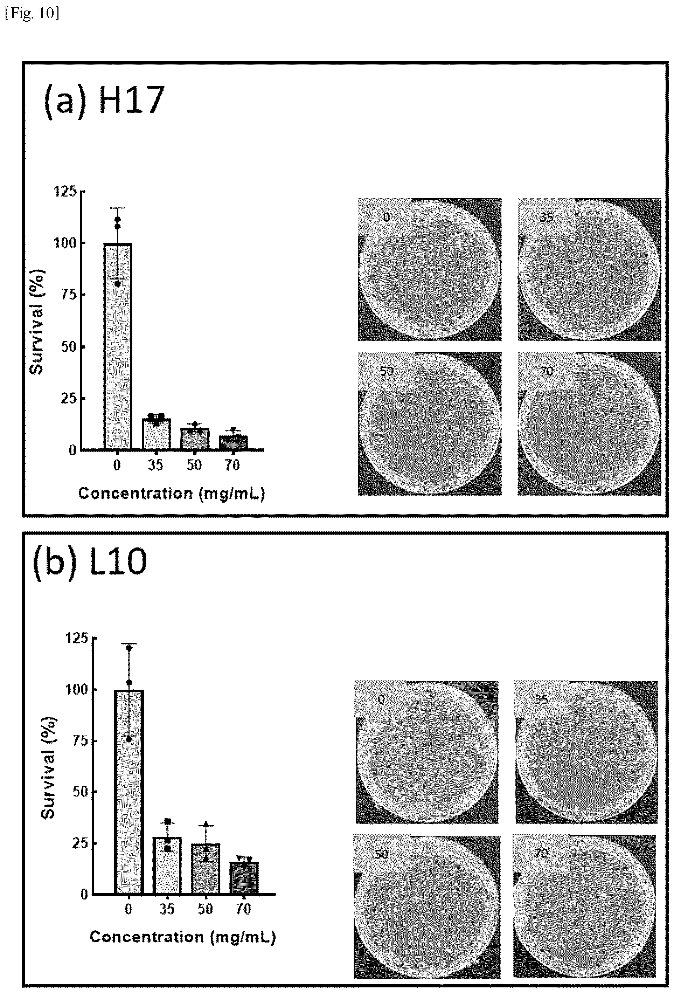

FIG. 10 shows graphs and images of confirming the antibacterial activity of PHA nanoparticles synthesized from Example 1 against *E. coli,* which are results for (a) H17 and (b) L10.

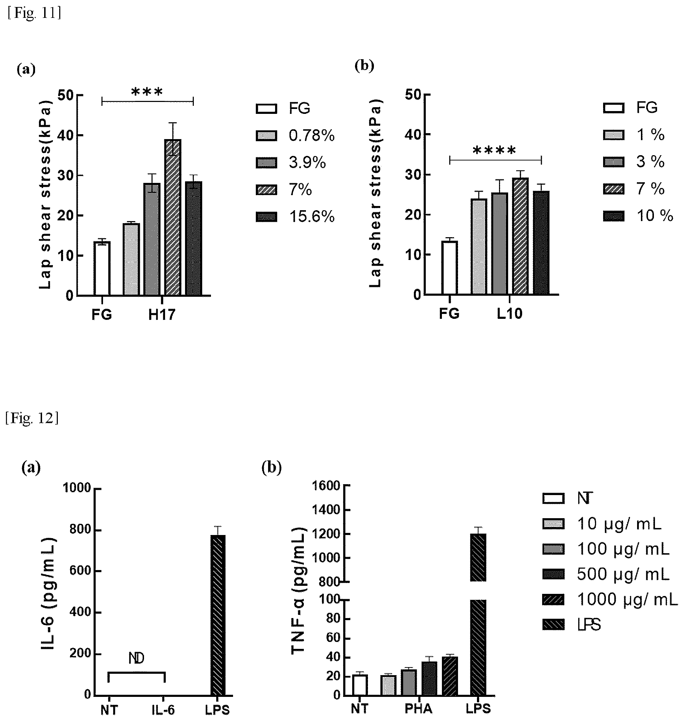

FIG. 11 shows results of evaluating tissue adhesion of PHA nanoparticles synthesized from Example 1, which are results for (a) fibrin glue (FG) and H17 and (b) fibrin glue (FG) and L10.

FIG. 12 is a graph showing results of confirming the inflammatory response of PHA nanoparticles synthesized from Example 1, which is a graph showing the levels of TNF-$\alpha$ and (b) IL-6 cytokines.

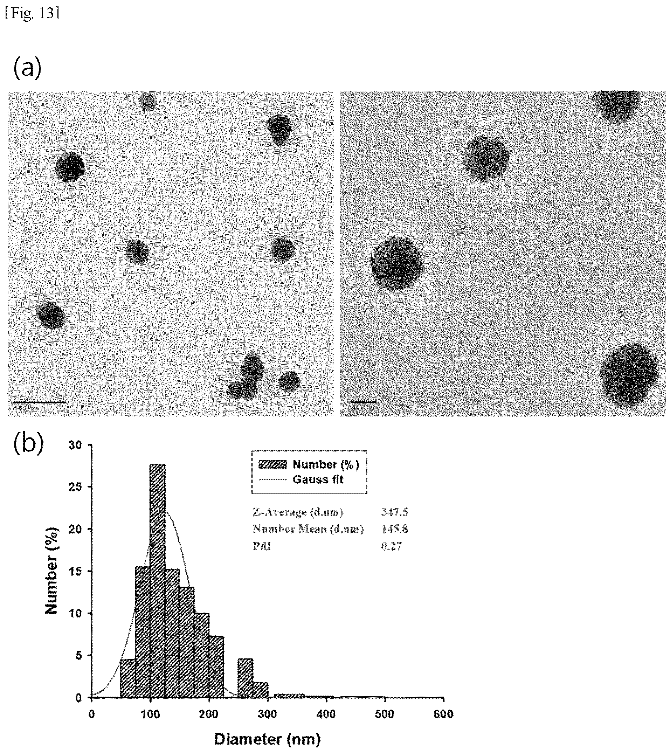

FIG. 13 is a graph showing (a) a TEM image and (b) a particle size distribution of LC-IO nanoparticles synthesized from Example 1.

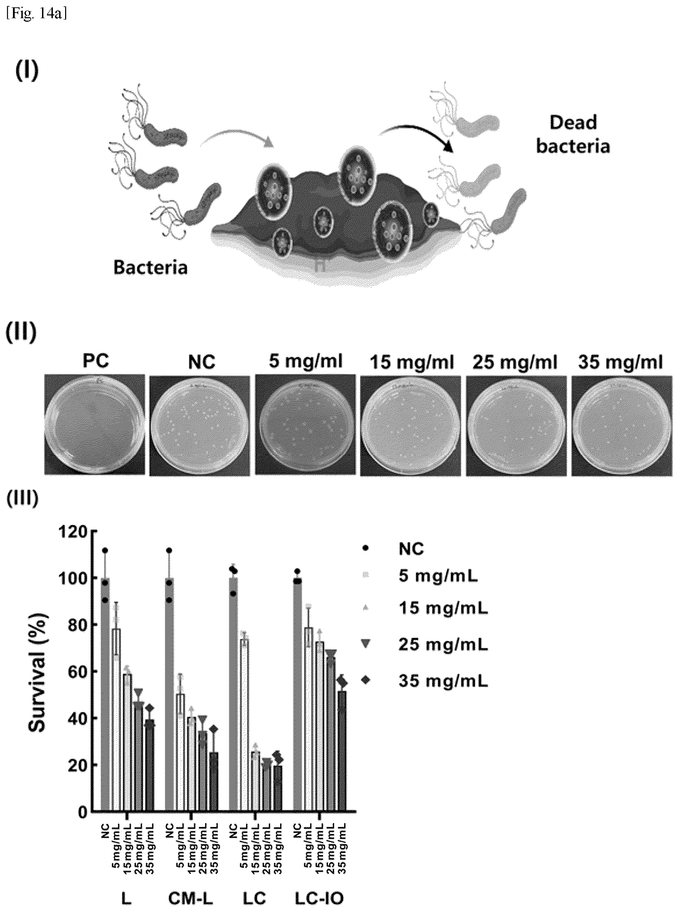

FIG. 14A shows results of evaluating the antibacterial properties of the LC-IO nanoparticles synthesized from Example 1, including (I) a schematic diagram of the antibacterial activity of the LC-IO nanoparticles, (II) images of colonies formed on an agar plate after treatment with LC-IO nanoparticles by concentration (NC is a negative control, PC is a positive control), and (III) a graph showing a survival rate (%) of *E. coli* using a colony forming unit test after treatment with levan, CM-levan, LC, and LC-IO nanoparticles by concentration.

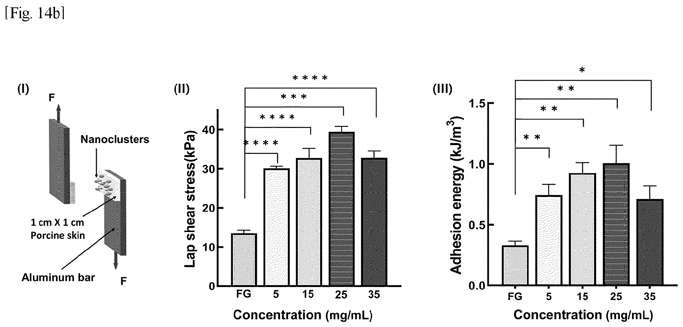

FIG. 14B shows mechanical properties of the LC-IO nanoparticles of Example 1 compared to fibrin glue, including (I) an *in vitro* lap shear test circuit diagram, (II) a graph showing maximum lap shear stress, and (III) a graph showing adhesion energy (n = 5).

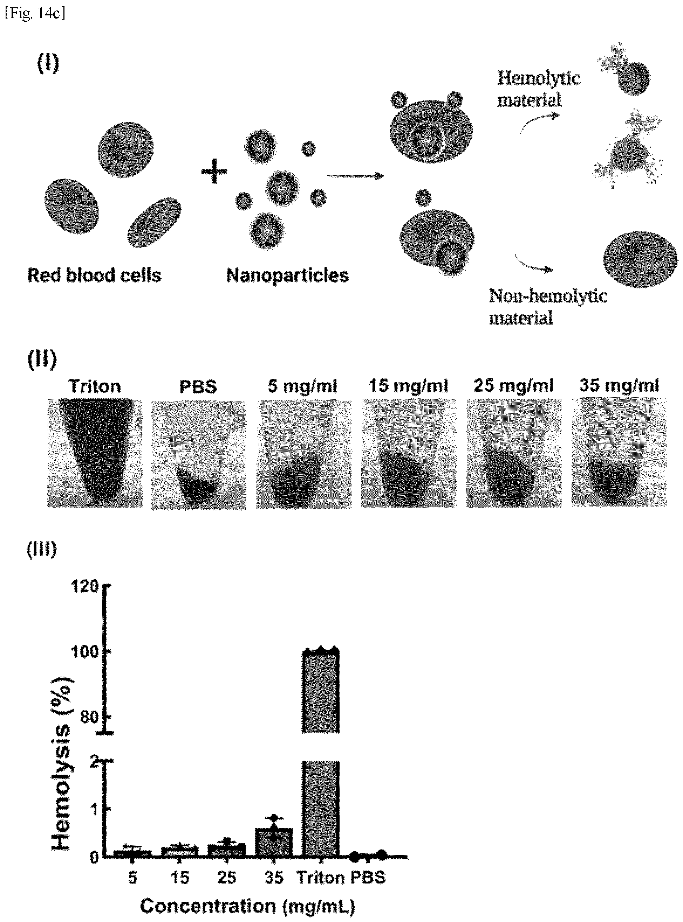

FIG. 14C shows results of evaluating blood compatibility of LC-IO nanoparticles synthesized from Example 1, including (I) a schematic diagram showing a hemolysis process, (II) images showing hemolysis after treatment with LC-IO nanoparticles, Triton (positive control), and PBS (negative control) by concentration, and (III) a graph showing a hemolysis rate (%) (n=3).

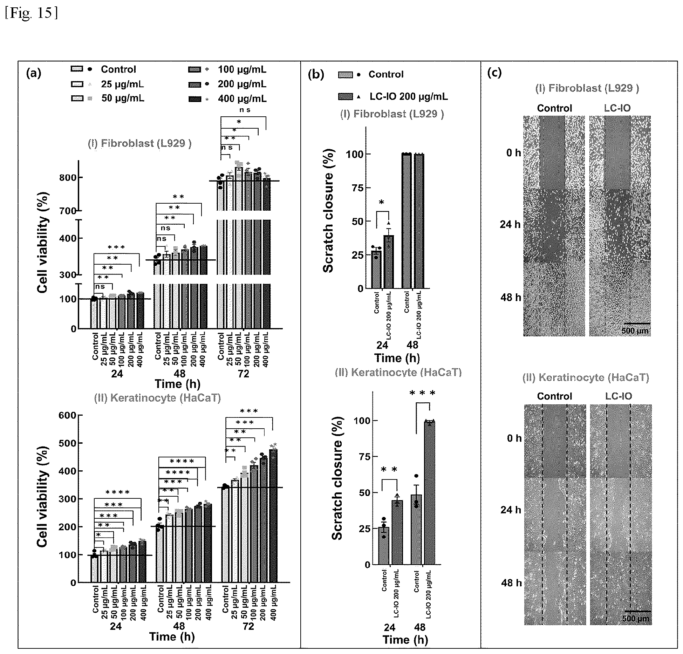

FIG. 15 shows results of evaluating the cell viability and migration of the LC-IO nanoparticles synthesized from Example 1, including (a) cell viability after treatment with LC-IO nanoparticles, (b) scratch area closure after treatment with 200 µg/ml of LC-IO nanoparticles, and (c) optical microscope images of cell migration.

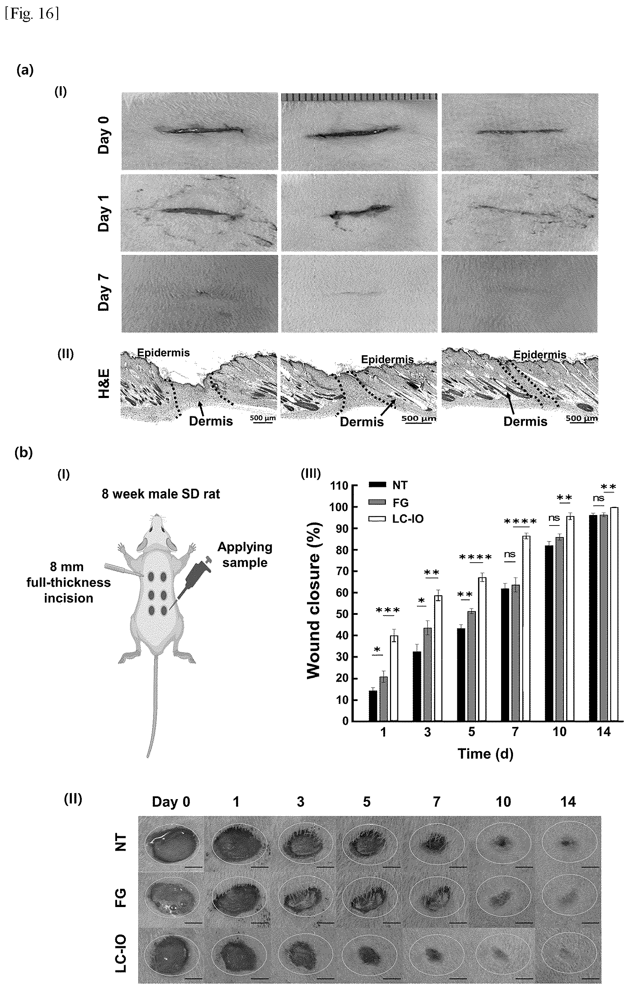

FIG. 16 shows results of evaluating *in vivo* wound healing of the LC-IO nanoparticles synthesized from Example 1, including (a) (I) images showing wound dynamics after treatment with LC-IO, fibrin glue FG (positive control), and an untreated wound (negative control) in order from the left for a one-dimensional wound (scale bar = 5 mm), and (II) H & E images 7 days after incision; (b) (I) a schematic diagram showing the incision site on the back of a rat for a two-dimensional wound, (II) images showing wound dynamics after treatment with LC-IO nanoparticles, fibrin glue FG (positive control), and an untreated wound (negative control), and (III) images showing wound closure (%) (n = 6).

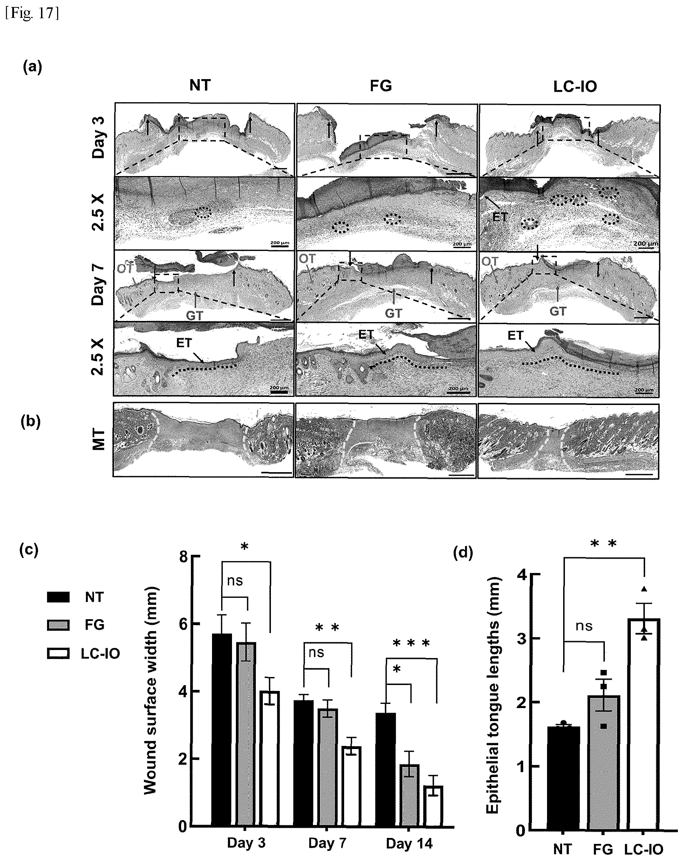

FIG. 17 shows results of histological examination of a wound cross-section of the LC-IO nanoparticles synthesized from Example 1, including (a) hematoxylin and eosin (H & E) stained 3 and 7 days after incision (the wound edge is indicated by a black arrow, newly formed blood vessels are indicated by a black dotted circle, and original tissue (OT) is indicated by a green arrow, granulation tissue (GT) is indicated by a red arrow, and epithelial tongue (ET) is indicated by a yellow dotted line), (b) an Masson's trichrome (MT) stained image on Day 14 (the yellow dotted line indicates granulation tissue), and graphs showing (c) wound surface widths on Day 7, and (d) epithelial tongue lengths (n = 3).

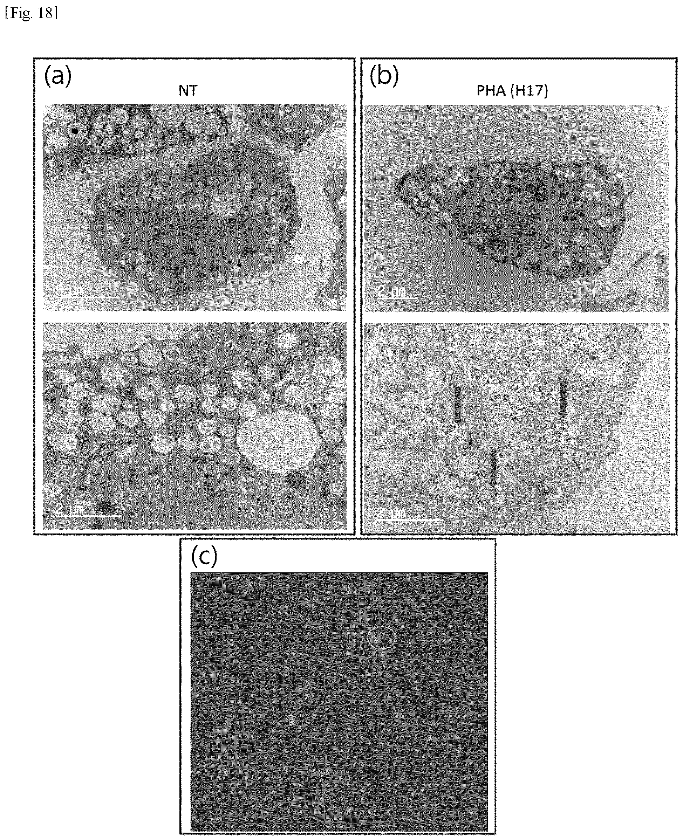

FIG. 18 shows results of confirming a state in which PHA nanoparticles (H17) are introduced into human dermal fibroblasts (HDFn) and decomposed into metabolite particles, including (a) human dermal fibroblasts in a H17 untreated group (NT) (upper: image of a single cell, lower: enlarged image of a single cell), (b) human dermal fibroblasts in a H17 treated group (PHA (H17)) (upper: image of a single cell, lower: enlarged image of a single cell), and (c) a transmission electron microscope (TEM) image of metabolite nanoparticles (indicated by yellow circles) in which PHA (H17) nanoparticles are decomposed inside human dermal fibroblasts.

**[Modes]**

Hereinafter, exemplary embodiments of the present disclosure will be described in detail with reference to the accompanying drawings. The exemplary embodiments of the present disclosure may be modified in various forms, and it should not be construed that the scope of the present disclosure is limited to exemplary embodiments to be described below. The exemplary embodiments will be provided for more completely explaining the present disclosure to those skilled in the art. Therefore, the shapes of components in the drawings are exaggerated to emphasize a clearer explanation.

Unless otherwise defined, all terms (including technical and scientific terms) used in the present specification may be used as the meaning which may be commonly understood by the person with ordinary skill in the art, to which the present disclosure pertains. Terms defined in commonly used dictionaries should not be interpreted in an idealized or excessive sense unless expressly and specifically defined.

Hereinafter, polymer nanoparticles of metabolites according to the present disclosure and a use thereof will be described in detail.

**Nanoparticles of metabolites and use thereof**

The present disclosure relates to polymer nanoparticles of metabolites (i.e., nanoparticles of polymerized metabolites), a cell activity promotion method using same, and a cell activity promotion composition including same. Particularly, the present disclosure relates to a method and a composition for promoting cell activities, such as adhesion between cells or between tissues, hemostasis, wound healing promotion, hair root regeneration activity, and antibacterial activity by the permeation of polymer nanoparticles of metabolites into cells.

The present disclosure demonstrated that when polymer nanoparticles of each metabolite are delivered into cells, the polymer nanoparticles are rapidly decomposed within the cells to supply nutrients, thereby exhibiting adhesion ability between cells or between tissues, hemostatic ability, wound healing promotion ability, hair root regeneration activity, and antibacterial activity. In particular, in the case of trauma or chronic wounds, it is impossible to deliver nutrients into cells until the nutrients are supplied from blood vessels. However, the polymer nanoparticles of the metabolites according to the present disclosure may adhere to cells with a large surface area to permeate into cells, and then rapidly supply the nutrients while releasing the metabolites through decomposition, and may exhibit a wound healing promotion effect by inhibiting the proliferation of infectious microorganisms while not involved in the proliferation of the infectious microorganisms within the cells. As such, a model with promoted wound healing may be a model in which wounds are healed quickly and have fewer scars. For example, in FIG. 18, it may be confirmed that PHA nanoparticles are introduced into animal cells and then decomposed into metabolites.

According to one aspect of the present disclosure, there is provided a cell activity promotion composition including, as an active ingredient, polymer nanoparticles of metabolites containing at least one selected from the group consisting of peptide bonds (amide bonds), ester bonds, and glycosyl linkages.

According to the present disclosure, there is provided a hemostatic composition including, as an active ingredient, polymer nanoparticles of metabolites containing at least one selected from the group consisting of peptide bonds (amide bonds), ester bonds, and glycosyl linkages.

Further, according to the present disclosure, there is provided a tissue adhesive composition including, as an active ingredient, polymer nanoparticles of metabolites containing at least one selected from the group consisting of peptide bonds, ester bonds, and glycosyl linkages.

Further, according to the present disclosure, there is provided a pharmaceutical composition for promoting wound healing including, as an active ingredient, polymer nanoparticles of metabolites containing at least one selected from the group consisting of peptide bonds, ester bonds, and glycosyl linkages.

Further, according to the present disclosure, there is provided an antibacterial composition including, as an active ingredient, polymer nanoparticles of metabolites containing at least one selected from the group consisting of peptide bonds, ester bonds, and glycosyl linkages.

Further, according to the present disclosure, there is provided a composition for hair root regeneration including, as an active ingredient, polymer nanoparticles of metabolites containing at least one selected from the group consisting of peptide bonds, ester bonds, and glycosyl linkages. Specifically, the composition for hair root regeneration according to the present disclosure may promote hair generation by promoting differentiation of stem cells that regenerate hair.

In the hemostatic composition, the tissue adhesive composition, the pharmaceutical composition for promoting wound healing, the composition for hair root regeneration or the antibacterial composition, the polymer nanoparticles of the metabolites may be proteins, polysaccharides and fats. Specifically, the polymer nanoparticles of the metabolites may be at least one selected from the group consisting of Pluronic F127, Tween20, Tween40, Tween80, gelatin, Poly(Hydroxyalkanoate) (PHA), Poly(Hydroxybutyrate) (PHB), polylactic acid (PLA), Poly(lactic-co-glycolic acid) (PLGA), levan, starch, amyloid, amyloid pectin, cellulose, chitin and chitosan. For example, the polysaccharides include sugars in which levan, starch, amyloid, amyloid pectin, cellulose, chitin, chitosan, and the like may be decomposed by enzymes in the body. In addition, among the polymer nanoparticles of the metabolites, PHA, PHB, PLA and PLGA include biomaterials used in a metabolic pathway.

Specifically, the polymer nanoparticles of the metabolites formed by peptide (amide) bonds may be exemplified as gelatin, the polymer nanoparticles of the metabolites formed by ester bonds may be exemplified as PHA, and the polymer nanoparticles of the metabolites formed by glycosyl linkages may be exemplified as levan.

The PHA may include repeating units derived from 4-hydroxybutyrate (4HB). In addition, the content of repeating units derived from 4-hydroxybutyrate (4HB) may be 0.1 to 60 wt% based on the total weight of the polyhydroxyalkanoate (PHA). In addition, the PHA may be a poly(3-hydroxybutyrate-co-4-hydroxybutyrate) copolymer. In addition, the PHA may have a molecular weight of 10,000 to 1,200,000 g/mol. Specifically, various types of PHA may be used, and any of crystalline, semi-crystalline or amorphous PHAs may be used. More specifically, in the semi-crystalline PHA, the content of repeating units derived from 4HB may be 8 to 20 wt%, and the molecular weight thereof may be 300,000 to 1,000,000. In addition, in the amorphous PHA (aPHA), the content of repeating units derived from 4HB may be 25 to 60 wt%, and the molecular weight thereof may be 500,000 to 1,000,000.

In the hemostatic composition, the tissue adhesive composition, the pharmaceutical composition for promoting wound healing, the composition for hair root regeneration or the antibacterial composition, the polymer nanoparticles of the metabolites may have a diameter of 300 nm or less, 200 nm or less or 100 nm or less. Specifically, the diameter may be 1 to 300 nm, 1 to 200 nm or 1 to 100 nm. More specifically, the diameter may be 50 to 300 nm, 50 to 200 nm or 50 to 100 nm. As described above, when the polymer nanoparticles of the metabolites according to the present disclosure have the diameter of 300 nm or less or the diameter within the limited range, introduction into cells and release of the metabolites occur more rapidly, thereby performing faster delivery of nutrients into cells, and also having an effect of inhibiting bacterial growth.

In the hemostatic composition, the tissue adhesive composition, the pharmaceutical composition for promoting wound healing, the composition for hair root regeneration or the antibacterial composition, the polymer nanoparticles of the metabolites may be hydrolyzed by enzymes or water within the cells after permeating into the cells to release the metabolites. Specifically, when the nanoparticles delivered into the cells are materials in which the metabolites are polymerized, the polymers of these metabolites may be decomposed by enzymes or water within the cells into metabolites serving as nutrients that promote the cell activity.

In the hemostatic composition, the tissue adhesive composition, the pharmaceutical composition for promoting wound healing, the composition for hair root regeneration or the antibacterial composition, the released metabolite may include (1) a metabolite having both a carboxyl group and a hydroxyl group, (2) a metabolite having both a carboxyl group and an amine group, (3) a metabolite having both an aldehyde group and a hydroxyl group, or (4) a metabolite having both a ketone group and a hydroxyl group.

In the hemostatic composition, the tissue adhesive composition, the pharmaceutical composition for promoting wound healing, the composition for hair root regeneration or the antibacterial composition, the released metabolite may be at least one selected from the group consisting of glucose, fructose, 3-hydroxybutyrate (3HB), 4-hydroxybutyrate (4HB), acetate, Botox and pyruvate.

The pharmaceutical composition mentioned in the present disclosure may be prepared by using pharmaceutically suitable and physiologically acceptable adjuvants in addition to the nanoparticles. As the adjuvants, excipients, disintegrants, sweeteners, binders, coating agents, expanding agents, lubricants, slip modifiers, or flavoring agents may be used.

A formulation form of the pharmaceutical composition may be granules, powders, tablets, coating tablets, capsules, suppositories, solutions, syrups, juice, suspensions, emulsions, drops, injectable liquids, or the like. For example, for formulation in the form of tablets or capsules, the active ingredient may be combined with an oral non-toxic pharmaceutically acceptable inert carrier, such as ethanol, glycerol, water, and the like. Further, if desired or necessary, suitable binders, lubricants, disintegrants and coloring agents may also be included as a mixture. The suitable binder is not limited thereto, but includes natural sugar such as starch, gelatin, glucose or beta-lactose, natural and synthetic gums such as corn sweetener, acacia, tragacanth or sodium oleate, sodium stearate, magnesium stearate, sodium benzoate, sodium acetate, sodium chloride, and the like. The disintegrant is not limited thereto, but includes starch, methylcellulose, agar, bentonite, xanthan gum, and the like. In the composition formulated as a liquid solution, the pharmaceutically acceptable carrier is suitable for sterilization and living bodies, and may be used with saline, sterilized water, ringer's solution, buffered saline, albumin injection solution, dextrose solution, maltodextrin solution, glycerol, ethanol, and a mixture of at least one of these ingredients, and if necessary, other general additives such as antioxidants, buffers, bacteriostatic agents, and the like may be added. In addition, the pharmaceutical composition may be formulated in injectable formulations such as an aqueous solution, a suspension, and an emulsion, pills, capsules, granules, or tablets by further adding a diluent, a dispersant, a surfactant, a binder, and a lubricant.

According to one embodiment of the present disclosure, the concentration of the polymer nanoparticles of the metabolites may be 0.1 to 100 $\mu$M, more preferably 10 to 50 $\mu$M, but is not limited thereto. Within the concentration range, the polymer nanoparticles of the metabolites not only exhibit excellent adhesion ability between cells or tissues, hemostatic ability, wound healing promotion ability, hair root regeneration activity, or antibacterial activity, but also exhibit no or very low cytotoxicity to renal cells.

Another aspect of the present disclosure provides a cell activity promotion method, including treating or administering the cell activity promotion composition according to the present disclosure to a subject other than a human or to a subject *in vitro*.

Further, the present disclosure provides a hemostatic method including treating or administering the hemostatic composition according to the present disclosure to a subject other than a human or to a subject *in vitro*.

Further, the present disclosure provides a tissue adhesion method including treating or administering the tissue adhesion composition according to the present disclosure to a subject other than a human or to a subject *in vitro*.

Further, the present disclosure provides a method for promoting wound healing including treating or administering the pharmaceutical composition for promoting wound healing according to the present disclosure to a subject other than a human or to a subject *in vitro*.

Further, the present disclosure provides an antibacterial method including treating or administering the antibacterial composition according to the present disclosure to a subject other than a human or to a subject *in vitro*.

Further, the present disclosure provides a method for promoting hair root regeneration or hair growth, including treating or administering the composition for hair root regeneration according to the present disclosure to a subject other than a human or to a subject *in vitro*. Specifically, the composition for hair root regeneration according to the present disclosure may promote hair generation by promoting differentiation of stem cells that regenerate hair.

As used herein, the term "subject" refers to a subject in need of prevention, alleviation or treatment for diseases, and more particularly, may mean mammals such as humans or non-human primates, mice, dogs, cats, horses and cattle, but is not limited thereto.

The dose of the hemostatic composition, the tissue adhesive composition, the pharmaceutical composition for promoting wound healing, the composition for hair root regeneration or the antibacterial composition according to the present disclosure will vary depending on the age, sex, and weight of a subject to be treated, a specific disease or pathological condition to be treated, the severity of the disease or pathological condition, a route of administration, and the judgment of a prescriber. The dose based on these factors is determined within a level of those skilled in the art, and in general, the dose is in the range of 0.01 mg/kg/day to about 2000 mg/kg/day. A more preferable dose is 0.1 mg/kg/day to 1000 mg/kg/day. The administration may be performed once a day or several times a day. The dose does not limit the scope of the present disclosure in any aspect.

The hemostatic composition, the tissue adhesive composition, the pharmaceutical composition for promoting wound healing, the composition for hair root regeneration or the antibacterial composition according to the present disclosure may be administered to mammals such as mice, livestock, and humans via various routes. All methods of administration may be expected and for example, the pharmaceutical composition may be administered by oral, rectal or intravenous, intramuscular, subcutaneous, intrauterine dural or cerebrovascular injection.

In the present disclosure, the hemostatic composition, the tissue adhesive composition, the pharmaceutical composition for promoting wound healing, the composition for hair root regeneration or the antibacterial composition may further include any compound or natural extract known to have the activity in which safety has already been verified in order to increase and reinforce adhesion ability between cells or tissues, hemostatic ability, wound healing promoting ability, and antibacterial activity, in addition to the active ingredient.

Further, the present disclosure provides a food composition or health functional food composition for hemostasis, tissue adhesion, wound healing promotion, hair root regeneration, or antibacterial purposes, including polymer nanoparticles of metabolites as an active ingredient.

The food or health functional food composition may further include an additive selected from the group consisting of flavoring agents, aromas, colorants, fillers, stabilizers, natural carbohydrates, nutrients, vitamins, thickeners, pH adjusters, preservatives, and mixtures thereof.

The food composition of the present disclosure includes all forms, such as functional foods, nutritional supplements, health foods, and food additives. The type of food composition may be prepared in various forms according to conventional methods known in the art.

For example, as the health food, the composition itself may be prepared in the form of tea, juice, and drinks to be drunk, or taken by granulation, encapsulation and powder. In addition, the functional food may be prepared by adding the extract to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc. In order to be used in the form of food additives, the composition of the present disclosure may be prepared and used in the form of powders or concentrates.

The preferred content of the polymer nanoparticles of the metabolites in the food composition of the present disclosure may be contained in the range of 0.001 to 50%, preferably 0.01 to 30%, based on the total weight of the food composition.

In an exemplary embodiment of the present disclosure, the health functional food composition of the present disclosure may be prepared in general formulations, such as tablets, pills, granules, powders, liquids, hard capsules, soft capsules, etc., and may be prepared in any form such as porridge, bread, beverages, bars, chocolate, cookies, tea, drinks, vitamin composite, meat, sausage, candy, noodles, jelly, etc.

In order to prepare various formulations or forms as described above, food acceptable carriers or additives such as the above-mentioned excipients may be used, and may be used with any carrier or additive known to be usable in the art in the preparation of the formulations or forms to be prepared.

Further, the present disclosure provides a feed composition for hemostasis, tissue adhesion, wound healing promotion, hair root regeneration, or antibacterial purposes, including polymer nanoparticles of metabolites as an active ingredient.

When the polymer nanoparticles of the metabolites of the present disclosure are provided in the form of the feed composition, the feed composition may additionally include known feed supplements, food additives, or feed additives, and may be prepared in the form of fermented feed, mixed feed, pellets, silage, etc.

The above description just illustrates the technical spirit of the present disclosure using an exemplary embodiment and various changes and modifications may be made by those skilled in the art to which the present disclosure pertains without departing from an essential characteristic of the present disclosure. Accordingly, the embodiments described in the present disclosure are not intended to limit the technical spirit of the present disclosure, but describe the present disclosure and the technical spirit of the present disclosure is not limited by these embodiments. The protective scope of the present disclosure should be construed based on the following claims, and all the techniques in the equivalent scope thereof should be construed as falling within the scope of the present disclosure.

Hereinafter, the present disclosure will be described in more detail through Examples.

## Materials

Table 1 below describes sample names and information on PHA used in the present disclosure.

[Table 1]

| Sample Name | 4HB(%) | Mw | n |
|---|---|---|---|
| L10 | 8.7 | 390k | 1.439 |
| H17 | 17 | 687k | 1.459 |
| aPHA | 48 | 800k | 1.454 |

## Example 1. Confirmation of wound healing promotion effect

### (1.1) Synthesis of poly(hydroxyalkanoate) (PHA) nanoparticles

Re-ground or pelletized PHA raw materials (L10 8.7% 4HB and Mw 390 kDa, H17 17% 4HB and $M_W$ 687kDa, aPHA 48% 4HB and Mw 800 kDa) were dissolved in chloroform at a concentration of 0.4 wt%. Subsequently, the generated PHA solution was extruded into an SDS aqueous solution (0.3 wt%) through an SPG hollow tube membrane to form an oil-in-water emulsion. An emulsifying device (IMK-40, Emtek, Korea) was purged with argon gas and pressurized to allow a dispersed phase to pass through pores. The applied pressure is inversely proportional to the pore size of the membrane to maintain the ratio of the applied pressure to a critical transfer pressure (Pc) of 230 kPa for each membrane with a 0.3 μm pore size [Coombs OBrien, J., et al., Continuous production of cellulose microbeads via membrane emulsification. ACS Sustainable Chemistry & Engineering, 2017.5(7): p. 5931-5939.], and a blade-type stirrer was rotated continuously at 200 rpm.

In a solvent evaporation method, chloroform within droplets was removed using a rotary evaporator. The emulsion was transferred to a 1 L round bottom flask and then evaporated at 57°C and 150 mbar for 3 hours. The rotary evaporator was rotated at 50 rpm during the entire evaporation process.

The PHA particles were washed five times with deionized water using a benzoylated cellulose dialysis tube (2,000 NMWCO, Sigma-Aldrich), collected by centrifugation at 2,000 rpm for 2 hours, and then the collected PHA nanoparticles were stored at room temperature until used.

(1.2) Synthesis of gelatin nanoparticles (GNPs)

Gelatin nanoparticles were prepared using a nano-precipitation method as previously reported [Lee, E.J., S.A. Khan, and K.H. Lim, Gelatin nanoparticle preparation by nanoprecipitation. J Biomater Sci Polym Ed, 2011.22(4-6): p. 753-71.]. Briefly, gelatin (125 mg) was dissolved in 10 ml of deionized water at 60°C. 5 ml of the gelatin solution was added dropwise to 40 ml of an ethanol solution of Pluronic F-127 (2%, w/v) while continuously stirring (mass ratio of emulsifier/gelatin = 32:1). After 15 minutes, 150 μl of a glutaraldehyde solution (5%, w/v) was added and the solution was stirred for 12 hours to crosslink the particles. The particles were purified through two cycles of centrifugation at 5000 rpm for 15 minutes and redispersion in deionized water. Finally, the synthesized gelatin nanoparticles were redispersed in 3 ml of water.

(1.3) Synthesis of levan-catechol-iron oxide nanoparticles (LC-IO) nanoparticles

(1.3.1) Synthesis of levan-catechol composite (LC)

A levan-catechol composite was synthesized by carbodiimide-promoted conjugation between the amine group of dopamine and the carboxyl group introduced into the carboxymethylated levan (CM-L) of Preparation Example 1. The CM-L (0.5 g) was dissolved in deionized water (100 mL), and then NHS (287.5 mg) and EDC (479.5 mg) were added to the solution to obtain a reaction solution. The reaction solution was stirred for 1 hour, and then added with dopamine (383 mg) to be adjusted to pH 5. The reaction solution adjusted to pH 5 was mixed and reacted at 500 rpm at room temperature under argon for 12 hours or more. After the reaction was completed, the reaction solution was dialyzed against acidic distilled water (pH 5.5) for two days and deionized water for 4 hours at 4°C, and then freeze-dried to synthesize a levan-catechol composite (LC).

(1.3.2) Synthesis of hydrophobic iron oxide nanoparticles (IO-NPs)

For the preparation of hydrophobic-coated IO-NPs, 1,2-hexadecanediol (1.95 g), oleic acid (1.65 mL), and oleylamine (2.85 mL) were dissolved in 25 mL of benzyl ether. Then, the mixture was reacted under vacuum at 60°C for 30 minutes. Thereafter, the temperature was raised to 200°C under nitrogen purging and the mixture was stored for 1 hour, and then refluxed at 290°C for 1 hour. After all reactions were completed, the IO-NPs solution was cooled and precipitated with 20 mL of cold ethanol. The prepared hydrophobic IO-NPs (in the range of 7 to 12 nm) were recovered through centrifugation (8,000 rpm, 6 min) and redispersed in hexane.

(1.3.3) Synthesis of levan-catechol-iron oxide (LC-IO) nanoparticles

Levan-catechol-iron oxide nanoparticles (LC-IO) were prepared using an electric sprayer (eS-robot system, NanoNC, SEOUL, Korea). The levan-catechol (LC) polymer solution for electrospray was prepared by dissolving 0.5 wt% LC (the levan-catechol composite synthesized in 1.3.1 above) in DMSO containing 0.3 mL of hydrophobic IO-NPs (10 mM Fe) in hexane, and then the phase-separated solution was sonicated in a water bath for 1 hour. Thereafter, the mixture was electrosprayed into an aluminum bath containing distilled water with magnetic stirring at a voltage of 23 kV, a flow rate of 5 μL/min, and a distance of 12 cm between the tip of a 23-gauge needle and a collector bath. The sprayed nanoparticle product was collected using a neodymium magnet and then dispersed in 15% DMSO for long-term storage. The synthesized LC-IO nanoparticles were confirmed to have particle sizes of 50 to 200 nm or 100 to 150 nm (see FIG. 13).

(1.4) Experiments and Results

All animal studies were performed in accordance with national regulations and approved by the Institutional Animal Care and Use Committee of POSTECH (IRB No. POSTECH-2022-0004, & POSTECH-2022-0116) with respect to Sprague Dawley rats (SD 150-200 g, male, 7 weeks old). To evaluate the wound healing properties of the synthesized PHA, gelatin, and LC-IO nanoparticles, Sprague Dawley rats were anesthetized using isoflurane, the back was shaved, and three circular incisions with a diameter of 8 mm were made on both sides of the back of each rat using a biopsy punch. The incision area was rapidly sealed with 25 μl of a nanoparticle solution (25 mg/ml) or fibrin glue (positive control), an untreated incision area was used as a negative control, and the back of the rat was covered with a Tegaderm transparent dressing film. Thereafter, the nanoparticle solution was sterilized under ultraviolet light for 2 hours. Wounds were photographed on days 0, 1, 3, 7, 10, and 14, and a relative wound closure rate (%) was calculated using Equation 1:

[Equation 1]

$$\text{Wound closure } \% = \frac{A_{to} - A_{tn}}{A_{to}} \times 100 \%.$$

In Equation 1, $A_{to}$ and $A_{tn}$ were the areas of the wound on day 0 and day n (n = 1, 3...).

Referring to FIG. 1, the *in vivo* wound healing properties of PHA, gelatin (GNP), and LC-IO nanoparticles were evaluated, and as a result, it was shown that wound healing was significantly accelerated compared to negative and positive controls. Here, it was determined that the nanoparticles served to directly supply nutrients to the wound area before angiogenesis occurred. For PHA nanoparticles with a high ratio of 4HB, the wound closure rate increased and the polymer became more amorphous. In addition, referring to the images of PHA particles (aPHA, L10, and H17) in FIG. 1B, it may be confirmed that hair generation was promoted along with wound healing, which was determined that the PHA nanoparticles promoted the hair generation by promoting the differentiation of stem cells that regenerate hair while introduced into the cells.

## Example 2. Confirmation of chronic wound treatment and antibacterial effects through PHA nanoparticles

(2.1) *In vitro* antibacterial effect assay methods

(2.1.1) CFU assay

The provided H17 and L10 (hereinafter, information described in Table 1 below) were stored in a refrigerator (4°C) and used if necessary. The experiment was performed by selecting treatment concentration of nanoparticles to be 17.5 mg/mL (total 1.75 mg nanoparticles in 100 μL) to treat the same amount of nanoparticles (70 mg/mL, total 1.75 mg nanoparticles in 25 μL) used in an *in vivo* rat wound healing experiment at Pohang University of Science and Technology. 70, 35, 17.5 and 8.75 mg/mL of H17, L10 nanoparticles and suspensions of Gram-positive bacteria MRSA (Methicillin-resistant Staphylococcus aureus) or Gram-negative bacteria PA (Pseudomonas aeruginosa) (PBS bacterial non-growth conditions, total volume 100 μL) were incubated at 37°C for 24 hours, and then CFU assay was performed to confirm an antibacterial effect. In addition, the antibacterial effect was confirmed by CFU assay after incubating H17, L10 nanoparticles at 17.5, 8.75, 4.38, and 2.17 mg/mL and MRSA or PA suspensions (TSB media bacterial growth conditions, total volume 100 μL) at 37°C for 24 hours. To prevent contamination by bacteria, all experiments for confirming the antibacterial effect were conducted inside a clean bench, and were sealed when sent outside the bench.

(2.1.2) Live & dead assay

17.5, 8.75, 4.38 and 2.17 mg/mL of H17, L10 nanoparticles and MRSA or PA suspensions were incubated at 37°C for 24 hours. Thereafter, each sample was stained with SYTO-9 and PI (LIVE/DEAD Viability/Cytotoxicity Kit, Thermo fisher scientific) and photographed by confocal laser scanning microscopy.

(2.1.3) Biofilm eradication assay

MRSA and PA suspensions (OD600: 0.2) were incubated in TSB media (containing 1% sucrose) in a 24-well plate (at 37°C and 100% humidity conditions) for two days to form a biofilm. The biofilm was washed three times with PBS to remove planktonic bacteria and the media. The formed biofilm was treated with 17.5 mg/mL of H17, L10 nanoparticles, and the biofilm biomass was measured at intervals of 8, 16, and 24 hours (the biofilm was stained with 0.5% crystal violet, and then the absorbance of the solution completely dissolved in ethanol was measured at 550 nm using a microplate).

<u>(2.2) Method for analyzing *in vivo* chronic wound healing effect</u>

(2.2.1) Diabetes model (STZ-induced type 1 diabetes) in chronic wound healing effect experiment

The treatment concentration of nanoparticles was selected to be 17.5 mg/mL (100 μL) to treat the same amount of nanoparticles (70 mg/mL, 25 μL) used in an *in vivo* rat wound healing experiment at Pohang University of Science and Technology, and then the experiment was performed. ICR 6-week-old male mice were acclimatized for 1 week, and then intraperitoneally injected with streptozotocin (STZ) at a concentration of 100 mg/kg total twice at two-day intervals to induce type 1 diabetes (mice with fasting blood sugar levels of 300 mg/dL or higher were selected for the experiment). After hair removal, a full-thickness wound was generated using an 8 mm biopsy punch, and then the mice were divided into groups administered with H17 nanoparticles (70 mg/mL, 25 μL) once and at intervals of 2, 4, and 6 days, and thereafter, Tegaderm attachment and bandage were performed. The Tegaderm attachment and bandage were performed by administering the same concentration of L10 nanoparticles once. The wound tissue was identified and the area was measured every two days, and after the experiment was completed, the wound tissue was sampled and subjected to H & E and MT staining to observe a histological appearance.

(2.2.2) Infectious diabetes model (STZ-induced type 1 diabetes & MRSA or PA infection) in chronic wound healing effect experiment

6-week-old male mice were acclimatized for 1 week, and then intraperitoneally injected with streptozotocin (STZ) at a concentration of 100 mg/kg total twice at two-day intervals to induce type 1 diabetes (mice with fasting blood sugar levels of 300 mg/dL or higher were selected for the experiment). After hair removal, a full-thickness wound was created using an 8 mm biopsy punch, and then the wound area was administered with an MRSA or PA suspension ($10^8$ CFU/mL, 20 μL), and then attached with Tegaderm. After infection induction for two days, H17, L10 nanoparticles (70 mg/mL, 25 μL) were administered once, and Tegaderm attachment and bandage were performed. The wound tissue was identified and the area was measured every two days, and after the experiment was completed, the wound tissue was sampled and subjected to H & E and MT staining to observe a histological appearance.

<u>(2.3) Results of *in vitro* antibacterial effect experiment</u>

(2.3.1) Results of CFU assay and Live & dead assay

The results of the antibacterial effect of H17, L10 nanoparticles on MRSA and PA experimented above were as follows.

First, MRSA bacteria at a concentration of approximately $10^6$ CFU/mL were incubated with H17, L10 nanoparticles at concentrations of 70, 35, 17.5, and 8.75 mg/mL in a PBS environment for 24 hours, and then the antibacterial effect was measured through CFU assay. Referring to FIG. 2A, it could be confirmed that the antibacterial effects of 4.0, 3.8, 3.2, and 1.7 log CFU/mL were shown at concentrations of 70, 37.5, 17.5, and 8.75 mg/mL of H17 nanoparticles, respectively, and the antibacterial effects of 2.8, 3.3, and 1.4 log CFU/mL were shown at concentrations of 70, 35, and 17.5 mg/mL of L10 nanoparticles, respectively.

To determine an effect of a high concentration of PHA nanoparticles on an MRSA colony formation process, CFU assay was performed by adding the MRSA suspension with the nanoparticles immediately before serial dilution. Referring to FIG. 2B, no antibacterial effect was shown even under high concentration nanoparticle conditions of 70 and 35 mg/mL. Based on the results, it is determined that the antibacterial effect is shown when the nanoparticles and bacteria are incubated together for a certain period of time (24 hours).

Thereafter, an *in vitro* antibacterial effect test was conducted under bacterial growth conditions similar to an *in vivo* environment (TSB media, 37°C conditions) and the amount of nanoparticles applied to actual wounds (70 mg/mL, total 1.75 mg nanoparticles in 25 μL, 17.5 mg/mL, total 1.75 mg nanoparticles in 100 μL). Referring to FIG. 2C, a control group that was not treated with a drug grew to a bacterial concentration of 8.7 log CFU/mL for MRSA and 9.9 log CFU/mL for PA, and when incubated for 24 hours with 17.5 mg/mL of H17, L10 nanoparticles, the antibacterial effect was shown at 1.7 log CFU/mL for MRSA and 0.9 log CFU/mL for 8.75 mg/mL of L10 nanoparticles, and no antibacterial effect was observed at other concentrations.

Referring to FIG. 2D, it may be confirmed that in the case of PA, no antibacterial effect is observed at any concentration including the highest concentration of H17, L10.

Accordingly, when the results through CFU assay were synthesized, the antibacterial effect of H17, L10 nanoparticles was somewhat shown under PBS conditions where bacteria could not grow (3.2, 1.4 log CFU/mL reduction at 17.5 mg/mL of H17, L10 nanoparticles, respectively, based on MRSA). However, in media conditions similar to an actual *in vivo* environment where bacteria may grow, an antibacterial effect of up to 1.7 log CFU/mL was shown in MRSA, but it was difficult to expect an infectious wound healing effect because a high concentration of bacteria of $10^7$ CFU/mL or higher still existed.

After a process of incubating nanoparticles and bacteria for 24 hours for CFU assay, aggregated white clumps (estimated to be several hundred micrometers in size) were formed. When bacteria and PHA nanoparticles were placed together, it was determined that it was necessary to confirm whether the metabolites were generated as a result of nanoparticle transformation (aggregation) or particle decomposition.

Live & dead assay was performed to qualitatively analyze the antibacterial effect of H17, L10 nanoparticles against MRSA and PA. After incubating the bacteria with nanoparticles at the same concentration as in the CFU assay for 24 hours, the Live & Dead assay was performed.

Referring to FIGS. 3A and 3B, red fluorescence of PI was generally observed when there was an antibacterial effect of about 3 to 4 log CFU/mL, and there was no difference between treatment with H17 and L10 at different concentrations (17.5, 8.75, 4.38, and 2.17 mg/mL) and an untreated group in both MRSA and PA (green fluorescence of SYTO-9 indicated live bacteria, and red fluorescence of PI indicated dead bacteria).

(2.3.2) Results of biofilm eradication assay

When actual *in vivo* wounds were infected with MRSA and PA, biofilms were formed, leading to chronic wounds. Accordingly, biofilm eradication assay was conducted to measure the biofilm eradication effect of H17, L10 nanoparticles on MRSA and PA biofilms. The results of the biofilm eradication assay of MRSA and PA were as follows.

The MRSA and PA biofilms incubated for two days were washed with PBS and incubated with 17.5 mg/mL of H17, L10 nanoparticles, and then the remaining biofilm biomass was measured by staining with crystal violet. Since there was a difference in the antibacterial effect depending on a nanoparticle treatment time in the previous antibacterial experiment, the biofilm biomass was measured after treatment for up to 24 hours (8, 16, and 24 hours).

Referring to FIGS. 4A and 4C, there was no difference in the amount of biomass between the H17, L10 nanoparticle treated group and the untreated group. Rather, it may be seen that the biomass slightly increased from about 2.1 to 2.3, which is determined that the biofilm eradication effect of the nanoparticles is not shown under the media conditions in which biofilms may grow, and thus the biomass increases as the incubation time passes. The results expressed as a % biomass also showed no difference from the untreated group.

FIGS. 4B and 4D correspond to representative photographs taken after staining the biofilm of each group with crystal violet. Consistent with the previous biomass measurement results, it may be confirmed that the stained color becomes darker as the incubation time passes.

(2.4) Results of *in vivo* chronic wound healing effect experiment

(2.4.1) Results of diabetes model (STZ-induced type 1 diabetes) assay in chronic wound healing effect experiment

The results of noninfectious diabetic wound healing were as follows.

The nanoparticle treatment concentration was administered once or multiple times (at intervals of 2, 4, or 6 days) as an amount (70 mg/mL, 25 $\mu$L per wound) used in an *in vivo* mouse wound healing experiment at Pohang University of Science and Technology. Referring to FIG. 5A, a higher wound healing effect was shown in the group administered with H17 nanoparticles once compared to the untreated group. In particular, 45% of wound healing was shown on Day 2, which showed a statistically significant difference from the untreated group (12%) ($p < 0.001$). Thereafter, on Day 4 and Day 6, 54% and 67% of wound healing were shown, respectively, which showed a statistically significant difference from the untreated group (21% and 48%) ($p < 0.01$, $p < 0.05$).

Based on the results, when the number of times of nanoparticle administration was increased, H17 nanoparticles were treated at intervals of 2, 4, and 6 days to confirm the effect on late wound healing, and then wound healing was observed. Referring to FIG. 5B, all three groups showed approximately 40% wound healing on Day 2, which showed high early wound healing similar to a single administration, but no late wound healing effect was observed with subsequent additional administrations. As a result, it may be confirmed that there is no statistically significant difference compared to a single administration.

Finally, the wound healing effects of a single administration of H17, L10 were compared. Referring to FIG. 5C, the L10 single-administration group showed wound healing of 15%, 42%, and 47% on Days 2, 4, and 6, respectively, which was a level that showed a statistically insignificant difference from the untreated group. When comparing the L10 single-administration group with the H17 single-administration group, there was a difference of 30% ($p < 0.001$) on Day 2, 12% on Day 4, and 20% on Day 6 ($p < 0.05$). Based on the results, it was shown that treatment with L10 nanoparticles showed no significant difference in the non-infected diabetic wound healing.

In addition, in the wound healing images of FIG. 6, it may be confirmed that hair generation was promoted along with the wound healing process over time, which was determined that the PHA nanoparticles promoted the hair generation by promoting differentiation of stem cells that regenerated hair while introduced into the cells.

To observe the histological characteristics of the wound, H&E staining and MT staining were performed to observe the tissue. In the H&E staining photographs of FIG. 7, histological changes that seemed to promote wound healing, including angiogenesis, were observed in the tissues of the H17 nanoparticle-treated group on Day 2 compared to the untreated group. On Day 6, not only a thick dermis structure but also a hair follicle structure was formed, and the outermost epidermis was generated, which indicated a high degree of wound healing.

The degree of collagen regeneration in wound tissue may be determined from the MT staining photographs in FIG. 7 (a part stained in blue was collagen-regenerated tissue). In the untreated group, no collagen regeneration occurred at all until Day 6 (tissue stained in red), but in the Day 6 photographs of the H17 nanoparticle-treated group, the dermis area began to be filled with collagen-regenerated tissue, and in the Day 12 photographs, collagen regeneration occurred in most tissues. In the Day 12 photographs of the L10 treated group, no significant difference was observed compared to the untreated group in both the H & E and MT staining photographs.

The results of non-infected diabetic wound healing showed that compared to the untreated group, in the H17 nanoparticle-treated group, the initial wound area decreased rapidly, and there was also a difference in the regeneration of structures that constituted skin tissue, such as the epidermis, dermis, hair follicles, and collagen, and in the L10 nanoparticle-treated group, there was no difference in wound area reduction and skin tissue regeneration compared to the untreated group.

(2.4.2) Results of infected diabetes model (MRSA infection diabetes, PA infected diabetes model) assay of chronic wound healing effect experiment

Results for wound healing from MRSA and PA infections were as follows.

The nanoparticle treatment concentration was administered once at the same concentration as in a non-infected wound model (70 mg/mL, 25 μL per wound). In the MRSA and PA infected wound photographs of FIGS. 8A and 9A, chronic wounds due to infection were able to be observed in both the untreated group and the H17, L10 nanoparticle-treated group. In both the MRSA and PA infection groups, wound healing hardly occurred when comparing Day 0 and Day 8, and white discolored skin tissue was observed due to the formation of MRSA and PA biofilms and the inflammatory response to infection.

In MRSA-infected diabetic mice, a nanoparticle untreated group had a wound healing effect of approximately 18% based on Day 8, while the H17, L10 nanoparticle-treated group had a wound healing effect of approximately 12% (not statistically significant).

In PA-infected diabetic mice, the nanoparticle untreated group had a wound healing effect of approximately 17% based on Day 8, while the H17, L10 nanoparticle-treated group had a wound healing effect of approximately 13% (not statistically significant).

To observe the histological characteristics of infectious wounds, H & E staining and MT staining were performed to observe the tissue. In FIGS. 8C and 9C, neither the untreated group nor the H17, L10 nanoparticle-treated group showed wound healing at the end of the experiment (Day 8). In the H & E staining photographs, the epidermis, dermis, and hair follicle structures were not observed, and only the wound matrix and inflammatory tissue structures were observed. In the MT staining photographs, collagen regeneration was not shown, and most structures were observed as red-stained collagen-free structures.

In summary, the wound healing effect was observed by a single administration of H17, L10 in MRSA and PA infected diabetes models, and in both cases, no wound healing effect was observed compared to the untreated group, which was interpreted as follows. To fabricate an *in vivo* infection wound model, $10^8$ CFU/mL of bacteria were injected, and these bacteria grew additionally in the wound area for two days. Therefore, even if an antibacterial effect of 1.7 log CFU/mL was measured in an *in vitro* experiment, it is determined to be insufficient to be effective in chronic wound healing because a high concentration of bacteria still exists in the wound area.

In a diabetic infectious chronic wound model in which diabetes and bacterial infection were simultaneously induced, the condition of the mice rapidly deteriorated over time (weight loss and slowed reaction time and behavior), and almost died. In addition, considering that the tendency of wound healing was maintained until Day 8, the experiment was terminated on Day 8.

(2.5) Confirmation of *E. coli* growth inhibition effect

The antibacterial activity of the PHA synthesized from Example 1 above was confirmed using pathogenic E. coli.

Referring to FIG. 10, it may be confirmed that PHA nanoparticles inhibit the growth of pathogenic *E. coli,* and it may be confirmed that the effect becomes greater as the treatment concentration of PHA nanoparticles increases. Through this, it may be confirmed that the antibacterial effect of PHA nanoparticles is excellent.

### (2.6) Discussion of results

The results of the chronic wound treatment and antibacterial effect experiment using the aforementioned PHA nanoparticles may be summarized as follows.

When an *in vitro* antibacterial effect experiment was performed with 17.5 mg/mL H17, L10 nanoparticles for 24 hours under bacterial growth conditions similar to the *in vivo* environment (TSB media, 37°C conditions), the antibacterial effect was shown at 1.7 log CFU/mL for MRSA and 0.9 log CFU/mL for 8.75 mg/mL of L10 nanoparticles, and no antibacterial effect was shown at other concentrations. For PA, no antibacterial effect was shown under the performed experimental conditions.

When the same concentration of nanoparticles as in the CFU assay was incubated with bacteria for 24 hours and Live & dead assay was performed, PI (red fluorescence) generated when bacteria were killed was not observed (generally, red fluorescence was observed when there was an antibacterial effect of about 3 to 4 log CFU/mL).

When the remaining biofilm biomass was measured by measuring the absorbance after treating the biofilms of MRSA and PA with 17.5 mg/mL of H17, L10 nanoparticles for 8, 16, and 24 hours and then staining with crystal violet, the biofilm eradication effect was not observed at all concentrations.

An experiment for an *in vivo* chronic wound healing effect was conducted in a non-infected diabetes model and a MRSA, PA infected diabetes model, and in the non-infected diabetes model, the initial wound healing effect of H17 (45% healing on Day 2) was prominent.

Thereafter, the experiment was performed by dividing the H17 administration into 2, 4, and 6-day intervals and increasing the number of times of administration, but no difference was shown compared to a single administration. Based on this, it is determined that H17 nanoparticles play an important role in the early stage of wound healing.

When comparing the untreated group with the L10-administered group, there was no significant difference in wound healing.

The wound healing effect was observed in the MRSA and PA infected diabetes models by single administration of H17, L10 nanoparticles. In both cases, no wound healing effect was observed compared to the untreated group. In the *in vivo* experiment, the concentration of bacteria injected into the wound was $10^8$ CFU/mL, and additional bacteria grew in the wound area for two days. Even though the antibacterial effect of 1.7 log CFU/mL measured in the *in vitro* experiment was shown, it was determined to be insufficient to exhibit an effect on chronic wound healing because a high concentration of bacteria still existed in the wound area.

In conclusion, it is determined that the antibacterial effect of PHA nanoparticles is insufficient for use *in vivo* conditions, and among the H17, L10 nanoparticles, the H17 nanoparticles particularly had excellent initial healing ability of non-infected diabetic wounds. In addition, when an antibiotic with strong antibacterial activity was encapsulated into H17 nanoparticles to form a formulation, it is expected to have a great effect on the healing of infectious diabetic wounds due to a synergistic effect of the antibacterial effect and the early wound healing.

### Example 3. Confirmation of adhesive ability and biocompatibility of PHA nanoparticles

### (3.1) Evaluation of adhesive ability of PHA

The adhesion of the PHA nanoparticles synthesized from Example 1 above was evaluated.

The adhesion of PHA nanoparticles to a biological tissue was evaluated by measuring shear stress using a universal testing machine (Instron 5544, Norwood, MA, US), and the results were shown in FIG. 11. Specifically, porcine skin (stellen Medical, USA) was cut into 10 × 10 mm pieces, placed in 0.1 M PBS buffer (pH 7.4), and left at 37°C for 1 hour. The porcine skin that had been left was attached to an aluminum bar having a size of 10 × 100 mm using an instant adhesive (3M). Then, H17 and L10 as samples for each concentration (the concentration % shown in the graph represented wt%) and fibrin glue (FG) (Green Cross, Korea) as a positive control were applied to the surface of the porcine skin. Then, the aluminum bars attached with the porcine skin without applying the samples were overlapped and the two aluminum bars were fixed using clips. After the two fixed aluminum bars were incubated at 100% relative humidity and room temperature (RT) for 2 hours, the shear stress was measured using a 10 kN load cell at a cross head speed of 10 mm/min until the two aluminum bars were completely separated.

Referring to FIG. 11, it may be confirmed that the tensile strength of PHA nanoparticles is superior to that of fibrin glue, and thus the adhesion is superior. In addition, it may be confirmed that the tensile strength of L10 is superior to that of H17, and thus the adhesion of L10 is superior to that of H17.

(3.2) Confirmation of inflammatory response of PHA

The biocompatibility of the PHA nanoparticles synthesized from Example 1 above was confirmed.

RAW 264.7 mouse macrophage-like cells were incubated in fresh Welgene DMEM supplemented with FBS (10 vol%) and penicillin-streptomycin (1 vol%) in a humidified incubator containing 5% $CO_2$ at 37°C. The cells were seeded on a 96-well plate at a density of $2.0 \times 10^4$ cells per well (300 μL) and maintained in 5 % $CO_2$ for 4 hours at 37°C. After the cells had adhered to the plate, the culture medium (100.0 μL) was replaced with a PHA nanoparticle solution in DMEM to final concentrations of (1000, 500, 100, or 10 μg/mL), and the cells were incubated with the media alone to be used as a negative control (NT). As previously reported, when RAW 264.7 cells were stimulated with lipopolysaccharide (LPS) at 500 ng/mL, the optimal level of TNF-α was released without cytotoxicity. Therefore, the cells were selected as a positive control (Nayak, Kaur, & Buttar, 2016). After treatment for 24 hours, each supernatant was collected and stored at -20°C until additional use. The IL-6 and TNF-α concentrations in the culture supernatant were determined using a DuoSet ELISA kit according to the manufacturer's instructions. The concentrations of IL-6 and TNF-α were determined using standard curves, and the results were shown in FIG. 12.

Referring to FIG. 12, the amount of cytokines released after exposure to the samples for 24 hours may be confirmed. Lipopolysaccharide (LPS) was used as a positive control, wherein LPS is a major component of the cell wall of Gram-negative bacteria, is highly immunogenic, and is one of the best monocyte/ macrophage activators. It may be concluded that the PHA nanoparticles have low immunogenicity and excellent biocompatibility.

## Example 4. Confirmation of cell introduction and metabolite decomposition of PHA nanoparticles

To confirm whether PHA nanoparticles were introduced into cells and decomposed into metabolites to exhibit effects, the following experiment was conducted. First, human dermal fibroblasts (HDFn) were incubated in fresh Welgene DMEM supplemented with FBS (10 vol%) and penicillin-streptomycin (1 vol%) in a humidified incubator containing 5% $CO_2$ at 37°C. The cells were fixed with osmium before washing the structure with acetone, washed three times with anhydrous acetone, and then immersed for 3 days in a graduated Epon resin (Ted Pella Inc.) diluted in acetone (5, 15, 25, 50, 75, 100% (v/v)). After polymerization in an oven at 60°C for 24 hours, the resin was cut into 200 nm sections using a Leica EM UC7 (Germany) and applied to copper grids. All TEM images were taken using a transmission electron microscope (JEOL, JEM 1011 Tokyo, Japan). Next, after treating the cells with PHA nanoparticles for about 4 hours, the cells were observed using TEM. As a result, referring to FIG. 18, in the enlarged image (bottom) of FIG. 18B, the PHA nanoparticles were decomposed within the cells to be confirmed as sets of distributed black dots (indicated by arrows). Compared to the size of the PHA nanoparticles before decomposition of 100 to 200 nm, the size of the metabolites that were decomposed into metabolites and delivered into cells may be observed to be decomposed to a size of several tens of nm, which is much smaller than 100 to 200 nm (enlarged image in FIG. 18C). It may be clearly confirmed compared with the PHA nanoparticle untreated group (NT) in FIG. 18A.

## Example 5. Confirmation of antibacterial ability, adhesive ability, and blood compatibility of LC-IO nanoparticles

(5.1) Results of confirming antibacterial ability of LC-IO

Referring to FIG. 2A, colony formation assay was performed to confirm the OD value. It was shown that the number of colonies formed after treatment with levan and its derivatives was decreased as the concentration was increased. Levan-catechol (LC) showed a higher inhibitory effect than levan (L) and carboxymethylated levan (CM-L) at the same concentration in the range of 15 to 35 mg/mL. In the case of levan-catechol iron oxide nanoparticles (LC-IO), the inhibition of *E. coli* was evaluated using only colony forming units because the color of the nanoparticles interfered with the OD value. As the concentration increased, bacterial viability decreased, and at 35 mg/mL, 51% of bacteria survived. However, compared with other derivatives, the nanoparticles showed lower inhibitory ability, which may be due to a decrease in the material transfer rate of levan within bacterial cells. Considering the antibacterial activity, levan is a suitable candidate for wound healing applications. Antibiotics are very important in wound healing because the antibiotics help prevent or reduce bacterial infections. Wounds may provide an invasion point for bacteria, and the bacteria may proliferate and cause infections that slow or impede a healing process. Therefore, it is important to supply the wound area with nutrients necessary for promoting cell growth before the reconstruction of blood tissue, and it is preferred that sugar-based nutrition may be utilized by microorganisms.

(5.2) Results of confirming adhesive strength of LC-IO

Referring to FIG. 2B, the LC-IO nanoparticles exhibited the adhesive strength (30.12 ± 1.2 kPa) that was at least twice higher than that of commercially available fibrin glue (13.47 $\pm$ 1.8 kPa) even at a low concentration of 5 mg/mL. The adhesive strength increased until the nanoparticle concentration increased to reach a saturated state and then decreased until the limited adhesion surface available on a substrate was almost occupied. In addition, the nanoparticles exhibited higher adhesion energy and Young's modulus than fibrin glue and showed the same pattern as the shear strength.

(5.3) Results of confirming blood compatibility of LC-IO

Referring to FIG. 2C, hemolysis is an essential indicator of the blood compatibility of a biomaterial due to damage activity against red blood cells. Another issue has limited the application of inorganic nanoparticles that may cause hemolysis. In the present disclosure, to overcome these problems, fabricated organic nanoparticles (LC-IO) and effects thereof were evaluated using a hemolysis assay on red blood cells. The hemolysis rate was set to 5% as a basis. Even at a concentration of 35 mg/mL, LC-IO nanoparticles showed a hemolysis rate of less than 1%, which was confirmed that LC-IO nanoparticles were blood compatible and a promising material for biomedical applications.

**Example 6. Evaluation of biocompatibility and cell migration of LC-IO nanoparticles**

(6.1) Results of confirming biocompatibility of LC-IO nanoparticles

To evaluate a potential advantage when utilizing LC-IO nanoparticles in wound healing, first, cytotoxicity against a L929 mouse fibroblast cell line and an immortalized human keratinocyte cell line HaCaT was evaluated. Referring to FIG. 15A, both cell lines showed growth promotion when the nanoparticle concentration was increased from 25 $\mu$g/mL to 400 $\mu$g/mL. This demonstrates excellent biocompatibility of LC-IO nanoparticles, which dispelled concerns that may damage cell membranes through strong electrostatic interactions between nanoparticles and cells, which was one of the problems of inorganic nanoparticles.

(6.2) Results of evaluating cell migration of LC-IO nanoparticles

Since keratinocytes and fibroblasts need to migrate from the free edge of the wound during the wound healing process, an effect of LC-IO nanoparticles on cell migration was evaluated using cell scratching assay. Referring to FIGS. 15B and 15C, for HaCaT cells 24 hours after scratching, cells treated with 200 $\mu$g/mL of LC-IO nanoparticles showed an average recovery of 44.6%, which was significantly higher than untreated cells which had an average recovery of only 26%. After 48 hours, the cells treated with LC-IO showed a healing rate close to 99%, whereas untreated cells showed a low healing rate of 48.5%. This is probably determined because levan has been reported to play an important role in the activation of matrix metalloproteinase (MMP), which is secreted in an inactive (latent) form, which is important for tissue regeneration and remodeling, but is generally a required activation stage. In particular, the levan has been reported to activate MMP-9, which is expressed at the front edge of migrating keratinocytes during wound closure and plays a critical role in keratinocyte migration. Excellent cell proliferation and rapid migration data obtained for LC-IO nanoparticles may be very useful for wound healing applications.

**Example 7. Confirmation of *in vivo* tissue adhesion and wound healing ability of LC-IO nanoparticles**

(7.1) Results of confirming *in vivo* tissue adhesion of LC-IO

To evaluate an *in vivo* adhesive ability of LC-IO nanoparticles, a one-dimensional wound was created on the back of a SD rat model and adhered to nanoparticles. Referring to FIG. 16A, an image of the wound may be confirmed at a wound area treated with LC-IO nanoparticles, and it may be confirmed that the wound edges seamlessly adhered to each other. It is determined to be due to nanoparticles with the small sizes that allow the two tissues to perfectly adhere to each other and be aligned together. In contrast, in the wound area where fibrin glue was applied, an adhesive acted as a layer to prevent direct contact between the two tissues at the wound area. As a result of H&E and MT staining of LC-IO nanoparticles, it was confirmed that the LC-IO nanoparticles prevented the formation of a hard macroscopic barrier, so that the tissues were faster healed. In addition, as may be seen in FIG. 16A, the granulation tissue area of the LC-IO group was narrower than that of the other two groups.
In addition, the healing ability of LC-IO nanoparticles was evaluated using full-thickness incision of the SD rat model. Referring to FIG. 16B, skin photograph images were confirmed at various time points, showing the dynamics of the wound as a percentage of the healed area from the first day after incision based on Day 0. The LC-IO nanoparticles showed a relatively fast approach speed, and after 1 week, 86% of the wound was healed, while fibrin glue and untreated wound showed only 63% and 6% healing rates, respectively.

(7.2) Results of confirming *in vivo* wound healing ability of LC-IO

To further investigate the ability of LC-IO nanoparticles to promote wound healing, regenerated rat tissues were examined histologically at 3, 7, and 14 days after incision. Referring to FIG. 17A, as hematoxylin and eosin (H&E) staining results, it was shown that numerous new blood vessels were observed in a group treated with LC-IO nanoparticles for 3 days after incision, which indicated faster angiogenesis, which was important for wound repair. Newly formed blood vessels contribute to the formation of temporary granulation tissue, supporting the growth of new tissues and delivering nutrients and oxygen to the expanding tissue required to support the healing process. Without sufficient angiogenesis, it takes longer time to heal wounds and the wounds may be more susceptible to infection. In addition, the angiogenesis process helps remove waste products and dead cells from the wound area, thereby further aiding the healing process. In addition, wounds treated with the LC-IO nanoparticles showed faster re-epithelialization, and from Day 3, newly formed epithelial tongues were observed only in the group treated with LC-IO nanoparticles. It is determined to be because the nanoparticles promoted the growth and migration of keratinocytes, as shown in an *in vitro* experiment. Epithelialization is a very important stage in the wound healing process by including the migration and proliferation of epithelial cells to cover the wound surface and restore the protective barrier function of the skin. This process is important for preventing infection, promoting tissue regeneration, and maintaining homeostasis. Restoration of the epidermis through epithelialization is a characteristic of successful wound healing. The wound surface width in the nanoparticle group was shorter than that in the fibrin glue and untreated groups. Masson's trichrome (MT) is a type of histochemical staining used to determine the distribution of collagen in regenerated tissues, and keratin and muscle fibers were stained in red, collagen was stained in blue, the cytoplasm was stained in light red or pink, and cell nuclei was stained in dark brown or black. The darker the blue, the more mature collagen was contained. According to MT results on Day 14, it was shown that treatment with LC-IO nanoparticles not only resulted in closer wound, but also rapidly remodeled the granulation tissue into a more mature collagen content structure. A higher collagen index indicates a greater number of fibroblasts, which are responsible for producing and organizing collagen fibers, and this result is consistent with an *in vitro* experimental result showing that the use of LC-IO nanoparticles promotes the proliferation of fibroblasts.

**Claims**

1. A cell activity promotion composition comprising, as an active ingredient, polymer nanoparticles of metabolites containing at least one selected from the group consisting of peptide bonds, ester bonds, and glycosyl linkages.

2. The cell activity promotion composition of claim 1, wherein the cell activity promotion is at least one use selected from the group consisting of hemostasis, tissue adhesion, wound healing promotion, antibacterial, and hair root regeneration.

3. The cell activity promotion composition of claim 1, wherein the polymer nanoparticles of the metabolites are at least one selected from the group consisting of Pluronic F127, Tween20, Tween40, Tween80, gelatin, Poly(Hydroxyalkanoate) (PHA), Poly(Hydroxybutyrate) (PHB), polylactic acid (PLA), Poly(lactic-co-glycolic acid) (PLGA), levan, starch, amyloid, amyloid pectin, cellulose, chitin and chitosan.

4. The cell activity promotion composition of claim 1, wherein the polymer nanoparticles of the metabolites have a diameter of 300 nm or less.

5. The cell activity promotion composition of claim 1, wherein the polymer nanoparticles of the metabolites permeate into cells and are hydrolyzed by enzymes or water within the cells to release the metabolites.

6. The cell activity promotion composition of claim 5, wherein the released metabolite includes (1) a metabolite having both a carboxyl group and a hydroxyl group, (2) a metabolite having both a carboxyl group and an amine group, (3) a metabolite having both an aldehyde group and a hydroxyl group, or (4) a metabolite having both a ketone group and a hydroxyl group.

7. The cell activity promotion composition of claim 6, wherein the released metabolite is at least one selected from the group consisting of glucose, fructose, 3-hydroxybutyrate (3HB), 4-hydroxybutyrate (4HB), acetate, Botox and pyruvate.

8. A cell activity promotion method, comprising treating or administering the cell activity promotion composition according to any one of claims 1 to 7 to a subject other than a human or to a subject *in vitro*.

9. The cell activity promotion method of claim 8, wherein the cell activity promotion method is at least one method selected from the group consisting of a hemostasis method, a tissue adhesion method, a wound healing promotion method, an antibacterial method, and a hair root regeneration or hair growth promotion method.

[Fig. 1]

[Fig. 2]

(a) MRSA CFU assay Result (PBS Condition, 24-hour incubation)

(b) MRSA CFU assay Result (PBS Condition, 0-hour incubation)

(c) MRSA CFU assay Result (Media Condition, 24-hour incubation)

(d) PA CFU assay Result (Media Condition, 24-hour incubation)

[Fig. 3a]

[Fig. 3b]

[ Fig. 4 ]

[Fig. 5]

(a) **Diabetic wound area**
(%, **Untreated - H17** Single administration)

(b) **Diabetic wound area**
(%, **Untreated - H17** Multiple administration)

(c) **Diabetic wound area**
(%, **Untreated - H17, L10** Single administration)

(d) **Diabetic wound area**
(%, **All experimental groups**)

[Fig. 6]

[ Fig. 7 ]

Scale bar = 200um; EP = epidermal; DE = dermal; HD = hypodermis; M = wound matrix. Red arrow indicates the blood vessel formed in the cutaneous tissue.

[Fig. 8]

(a) MRSA Representative image of infected wound

(b) MRSA Infected wound area (%)

Day 0    Day 2    Day 4    Day 6    Day 8

Untreated

H17

L10

Day 0
Day 2
Day 4
Day 6
Day 8

Length (12 mm)

(c) MRSA Micrograph of infected wound

H&E

Untreated    H17    L10

Day 8

MT

Untreated    H17    L10

Scale bar = 200 um

[Fig. 9]

(a) **PA** Representative image of infected wound

(b) **PA** Infected wound area (%)

(c) **PA** Micrograph of infected wound

Scale bar = 200 um

EP 4 591 856 A1

[Fig. 10]

[Fig. 11]

(a)

(b)

[Fig. 12]

(a)

(b)

[Fig. 13]

(a)

(b)

| | |
|---|---|
| Z-Average (d.nm) | 347.5 |
| Number Mean (d.nm) | 145.8 |
| PdI | 0.27 |

[Fig. 14a]

[Fig. 14b]

[Fig. 14c]

[Fig. 15]

[Fig. 16]

(a)

(I)

(II)

(b)

(I)

8 week male SD rat

8 mm
full-thickness
incision

Applying
sample

(III)

(II)

[Fig. 17]

(a)

(b)

(c)

(d)

[Fig. 18]

**INTERNATIONAL SEARCH REPORT**

| | International application No. |
|---|---|
| | **PCT/KR2024/003942** |

### A. CLASSIFICATION OF SUBJECT MATTER

**A61K 9/14**(2006.01)i; **A61K 35/12**(2006.01)i; **A61K 31/715**(2006.01)i; **A61K 47/10**(2006.01)i; **A61K 47/02**(2006.01)i; **A61P 17/02**(2006.01)i

According to International Patent Classification (IPC) or to both national classification and IPC

### B. FIELDS SEARCHED

Minimum documentation searched (classification system followed by classification symbols)

A61K 9/14(2006.01); A61K 47/32(2006.01); A61K 9/70(2006.01); A61L 15/07(2006.01); A61L 15/12(2006.01); A61L 24/00(2006.01); A61L 24/10(2006.01); D04H 1/728(2012.01); D04H 13/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 대사물(metabolite), 나노 입자(nanoparticle), 세포 활성(activating cell)

### C. DOCUMENTS CONSIDERED TO BE RELEVANT

| Category* | Citation of document, with indication, where appropriate, of the relevant passages | Relevant to claim No. |
|---|---|---|
| X | KR 10-0791039 B1 (DAEGU GYEONGBUK INSTITUTE OF SCIENCE AND TECHNOLOGY et al.) 03 January 2008 (2008-01-03)<br>See abstract; claims 2 and 5; and paragraphs [0016], [0023]-[0024] and [0046]-[0049]. | 1-4,8-9 |
| Y | | 5-7 |
| Y | KR 10-2019-0059266 A (IMBED BIOSCIENCES INC.) 30 May 2019 (2019-05-30)<br>See paragraphs [0093]-[0094]. | 5-7 |
| A | KR 10-1005079 B1 (KUMOH NATIONAL INSTITUTE OF TECHNOLOGY INDUSTRY-ACADEMIC COOPERATION FOUNDATION et al.) 30 December 2010 (2010-12-30)<br>See entire document. | 1-9 |
| A | KR 10-2023-0026139 A (HANS BIOMED CORP) 24 February 2023 (2023-02-24)<br>See entire document. | 1-9 |

[✓] Further documents are listed in the continuation of Box C.  [✓] See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **25 June 2024** | **26 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2024/003942** |

### C. DOCUMENTS CONSIDERED TO BE RELEVANT

| Category* | Citation of document, with indication, where appropriate, of the relevant passages | Relevant to claim No. |
|---|---|---|
| A | KR 10-2017-0087245 A (INDUSTRY-ACADEMIC COOPERATION FOUNDATION GYEONGSANG NATIONAL UNIVERSITY) 28 July 2017 (2017-07-28)<br>See entire document. | 1-9 |

Form PCT/ISA/210 (second sheet) (July 2022)

**INTERNATIONAL SEARCH REPORT**
Information on patent family members

International application No.

**PCT/KR2024/003942**

| Patent document cited in search report | | | Publication date (day/month/year) | Patent family member(s) | | | Publication date (day/month/year) |
|---|---|---|---|---|---|---|---|
| KR | 10-0791039 | B1 | 03 January 2008 | None | | | |
| KR | 10-2019-0059266 | A | 30 May 2019 | CN | 109843343 | A | 04 June 2019 |
| | | | | EP | 3490622 | A1 | 05 June 2019 |
| | | | | EP | 3490622 | B1 | 06 September 2023 |
| | | | | EP | 4289910 | A2 | 13 December 2023 |
| | | | | EP | 4289910 | A3 | 13 March 2024 |
| | | | | KR | 10-2024-0025050 | A | 26 February 2024 |
| | | | | KR | 10-2637746 | B1 | 16 February 2024 |
| | | | | US | 11554194 | B2 | 17 January 2023 |
| | | | | US | 2018-0028713 | A1 | 01 February 2018 |
| | | | | US | 2024-0009342 | A1 | 11 January 2024 |
| | | | | WO | 2018-023021 | A1 | 01 February 2018 |
| KR | 10-1005079 | B1 | 30 December 2010 | KR | 10-2010-0045158 | A | 03 May 2010 |
| KR | 10-2023-0026139 | A | 24 February 2023 | None | | | |
| KR | 10-2017-0087245 | A | 28 July 2017 | KR | 10-1805781 | B1 | 07 December 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **COOMBS OBRIEN, J. et al.** Continuous production of cellulose microbeads via membrane emulsification. *ACS Sustainable Chemistry & Engineering*, 2017, vol. 5 (7), 5931-5939

- **LEE, E.J.** ; **S.A. KHAN** ; **K.H. LIM**. Gelatin nanoparticle preparation by nanoprecipitation. *J Biomater Sci Polym Ed*, 2011, vol. 22 (4-6), 753-71